# EUROPEAN PATENT APPLICATION

(11) **EP 4 692 343 A1**
(43) Date of publication of application: **11.02.2026**
(21) Application number: 24764061.8
(22) Date of filing: 27.02.2024
(51) Int. Cl.: C12N 15/11, C12N 5/10, C12N 15/12, C12N 15/13, C12N 15/63, C12P 19/34, C12P 21/02

(54) **CHINESE HAMSTER ARTIFICIAL CHROMOSOME VECTOR OR FRAGMENT THEREOF, AND MAMMALIAN-DERIVED CELL CONTAINING SAME**

(30) Priority: 28.02.2023 JP 2023029116
(71) Applicant: Trans Chromosomics, Inc., Yonago-shi, Tottori 683-8503 (JP)
(72) Inventor: OSHIMURA, Mitsuo, Yonago-shi, Tottori 683-8503 (JP); ENDO, Takeshi, Yonago-shi, Tottori 683-8503 (JP); HONMA, Kazuhisa, Yonago-shi, Tottori 683-8503 (JP); ABE, Satoshi, Yonago-shi, Tottori 683-8503 (JP)
(74) Representative: D Young & Co LLP
(86) International application number: PCT/JP2024/080020
(87) International publication number: WO 2024/181584

(57) **Abstract**

This application provides a novel means that is useful for stable production of biopharmaceuticals such as human antibodies. That is, this application provides: a Chinese hamster artificial chromosome that enables introduction of a foreign DNA of a target protein or the like in a site-directed manner into one or a plurality of DNA sequence insertion sites; a Chinese hamster artificial chromosome vector comprising a foreign DNA of a target protein or the like introduced into the Chinese hamster artificial chromosome; and a mammalian cell comprising the Chinese hamster artificial chromosome vector introduced thereinto.

## Description

### Technical Field

The present invention relates to a novel Chinese hamster artificial chromosome or a fragment thereof and a Chinese hamster artificial chromosome vector or a fragment thereof comprising such artificial chromosome and an exogenous DNA.

The present invention relates to a mammalian-derived cell comprising the Chinese hamster artificial chromosome vector or a fragment thereof.

The present invention further relates to a method for producing a useful protein or a human antibody using the cell.

### Background Art

An artificial chromosome vector is capable of introducing DNA that is larger than approximately 200 kb in general, such as a chromosome fragment of a mega-base scale, into a host cell. Accordingly, an artificial chromosome vector is used for, for example, human antibody production, drug metabolism testing, and preparation of a non-human animal that can be used as a disease model. As artificial chromosome vectors, a human artificial chromosome (HAC) vector, a mouse artificial chromosome (MAC) vector, and the like have heretofore been known. As the HAC vector, specifically, Patent Literatures 1, 2, and 3 report vectors derived from human chromosome 14 and human chromosome 21. As the MAC vector, Patent Literature 4 reports a vector derived from mouse chromosome 11, and Patent Literature 5 reports vectors derived from mouse chromosome 10 and mouse chromosome 16.

Many biopharmaceuticals, such as antibody medicine, are produced with the use of animal-derived cells, and a representative example thereof is Chinese hamster ovary cells (CHO cells). A gene encoding a target protein, such as an antibody that is effective for treatment of a disease, is introduced into CHO cells, so as to prepare CHO cells capable of expressing a target protein (e.g., an antibody). The thus-prepared CHO cells are subjected to large-scale culture, the resulting culture supernatant is purified, and a target protein (e.g., an antibody) can be thus obtained. In order to obtain a target protein (e.g., an antibody), specifically, it is necessary to obtain CHO cells that stably express a target protein (e.g., an antibody).

In order to produce a target protein such as an antibody or biopharmaceuticals with the use of CHO cells, it is necessary to obtain many clones of CHO cells and select clones having adequate properties. Among the selected clones, however, an expression level of the target protein is often lowered during passage culture of the cells. Accordingly, development of an artificial chromosome vector that can be obtained with the use of CHO cells as host cells has been demanded.

In the past, however, there has been no artificial chromosome derived from Chinese hamster.

### Prior art literatures

### Patent literatures

Patent literature 1: WO 2009/063722
Patent literature 2: WO 2004/031385
Patent literature 3: JP 2007-295860 A
Patent literature 4: JP Patent No. 5,557,217
Patent literature 5: WO 2019/177163

### Summary of Invention

It is an object of the present invention to provide a novel Chinese hamster artificial chromosome or a fragment thereof, a Chinese hamster artificial chromosome vector or a fragment thereof, and a mammalian-derived cell comprising the same that can be useful for production of useful biopharmaceuticals using CHO cells.

In order to attain the object, the present inventors prepared an artificial chromosome derived from Chinese hamster. More specifically, the present inventors developed a Chinese hamster artificial chromosome (hereinafter, it is often abbreviated to as "CHAC") capable of introducing an exogenous DNA of a target protein or the like in a site-directed manner into one or a plurality of DNA insertion sites. In addition, the present inventors succeeded in developing a method of stably expressing a useful protein, such as an antibody, via long-term passage culture by preparing a Chinese hamster artificial chromosome vector comprising an exogenous DNA introduced into its artificial chromosome (hereinafter, it is often abbreviated to as "CHAC vector").

The present invention has been completed based on the results of development described above. In short, the present invention includes the following features.
(1) A Chinese hamster artificial chromosome or a fragment thereof comprising a centromere and a telomere derived from a Chinese hamster chromosome, a part of the chromosome, and one or a plurality of DNA insertion sites.
(2) The Chinese hamster artificial chromosome or a fragment thereof according to (1), wherein the nucleotide size is 10 Mbp or smaller.
(3) The Chinese hamster artificial chromosome or a fragment thereof according to (1) or (2), wherein a sequence other than the DNA insertion site is derived from a Chinese hamster chromosome.
(4) The Chinese hamster artificial chromosome or a fragment thereof according to any of (1) to (3), wherein the DNA insertion site comprises at least one sequence selected from the group consisting of Bxb1 attP and Bxb1 attB sequences, φC31 attB and φC31 attP sequences, R4 attB and R4 attP sequences, TP901-1 attB and TP901-1 attP sequences, an FRT sequence, and a loxP sequence.
(5) The Chinese hamster artificial chromosome or a fragment thereof according to any of (1) to (4), which further comprises a reporter gene and/or a selection marker gene.
(6) A Chinese hamster artificial chromosome vector or a fragment thereof, which comprises an exogenous DNA introduced in a site-directed manner into the DNA insertion site of the Chinese hamster artificial chromosome according to any of (1) to (5).
(7) The Chinese hamster artificial chromosome vector or a fragment thereof according to (6), which comprises a plurality of exogenous DNAs.
(8) The Chinese hamster artificial chromosome vector or a fragment thereof according to (6) or (7), which further comprises another DNA insertion site in addition to one or a plurality of the DNA insertion sites.
(9) The Chinese hamster artificial chromosome vector or a fragment thereof according to any of (6) to (8), wherein the exogenous DNA is human DNA.
(10) The Chinese hamster artificial chromosome vector or a fragment thereof according to (9), wherein the exogenous DNA is a DNA of a gene or a chromosome locus.
(11) The Chinese hamster artificial chromosome vector or a fragment thereof according to (9) or (10), wherein the exogenous DNA is a gene encoding an immunoglobulin heavy chain and/or light chain or DNA of the gene.
(12) The Chinese hamster artificial chromosome vector or a fragment thereof according to any of (6) to (10), wherein the exogenous DNA is a gene selected from the group consisting of genes encoding polypeptides, such as cytokines, hormones, growth factors, nutritional factors, hematopoietic factors, coagulation or hemolysis factors, G protein-coupled receptors, and enzymes; a gene used for treatment of a diseases, such as tumor, muscular dystrophy, hemophilia, neurodegenerative disease, autoimmune disease, allergic disease, and genetic disease; and an immune system gene or DNA sequences, such as the T cell receptor (TCR) or the human leukocyte antigen (HLA).
(13) The Chinese hamster artificial chromosome vector or a fragment thereof according to any of (6) to (12), which further comprises a functional nucleic acid.
(14) The Chinese hamster artificial chromosome vector or a fragment thereof according to (13), wherein the functional nucleic acid is a gene that accelerates expression of an exogenous DNA.
(15) The Chinese hamster artificial chromosome vector or a fragment thereof according to (13), wherein the functional nucleic acid is a gene encoding a factor that is essential for mammalian cell survival.
(16) A mammalian-derived cell comprising the Chinese hamster artificial chromosome vector or a fragment thereof according to any of (6) to (15).
(17) The cell according to (16), which is transferred into a mammalian-derived cell and stably retained in the mammalian-derived cell over a long period of time.
(18) The cell according to (16), which is retained independently in a mammalian-derived cell or retained in the chromosome translocated to the mammalian-derived cell.
(19) The cell according to any of (16) to (18), wherein the mammalian-derived cell is a rodent-derived cell or a primate-derived cell.
(20) The cell according to (19), wherein the rodent-derived cell is a Chinese hamster-derived cell.
(21) The cell according to (19), wherein the primate-derived cell is a human-derived cell.
(22) The cell according to (16), which is deposited under CHO(hprt-/-)CHAC C4 #2 (NITE BP-03585).
(23) The cell according to (16), which is deposited under CHO(hprt-/-)CHAC C4 #4 (NITE BP-03586).
(24) The cell according to (16), which is deposited under CHO(hprt-/-)CHAC L14 #5 (NITE BP-03587).
(25) A method for producing a protein comprising: a step of production comprising culturing the cell according to any of (16) to (24) to produce a protein encoded by the exogenous DNA; and a step of collection comprising collecting the protein.
(26) The method according to (25), which improves the protein productivity by further inclusion of a functional nucleic acid.
(27) The method according to (26), wherein the functional nucleic acid is a gene that accelerates expression of an exogenous DNA in the cell.
(28) The method according to (26), wherein the functional nucleic acid is a gene encoding the factor that is essential for cell survival and only the cell having the artificial chromosome vector or a fragment thereof is able to survive.
(29) The method according to (25), which improves productivity of the protein by subjecting an exogenous DNA loaded on the Chinese hamster artificial chromosome to gene amplification.
(30) A kit for preparing a Chinese hamster artificial chromosome vector comprising: the Chinese hamster artificial chromosome or a fragment thereof according to any of (1) to (5); and a vector comprising one or a plurality of DNA insertion sites included in the Chinese hamster artificial chromosome.
(31) The kit according to (30), wherein the vector further comprises an exogenous DNA.
(32) A method for preparing a mammalian artificial chromosome or a fragment thereof comprising a centromere and a telomere derived from a mammalian chromosome, a part of the mammalian chromosome, and one or a plurality of DNA insertion sites, comprising:
   a step of constructing a vector for inserting DNA comprising constructing a vector comprising one or a plurality of the DNA insertion sites;
   a step of introducing a DNA insertion site comprising transfecting the vector for inserting DNA into a mammalian cell to introduce the DNA insertion site into the chromosome of the mammalian cell; and
   a step of fragmentation comprising transferring a chromosome comprising a DNA insertion site from a donor mammalian cell comprising the DNA insertion site after the step of introduction to a recipient mammalian cell by the microcell-mediated chromosome transfer (MMCT) method and fragmenting the chromosome.
(33) A method for preparing a mammalian artificial chromosome vector or a fragment thereof comprising:
   a step of constructing a vector for introducing an exogenous DNA comprising constructing a vector for introducing an exogenous DNA comprising one or a plurality of DNA insertion sites and an exogenous DNA; and
   a step of introducing a vector for introducing an exogenous DNA comprising transfecting the vector for introducing an exogenous DNA into a cell into which a mammalian artificial chromosome comprising a centromere and a telomere derived from a mammalian chromosome, a part of the chromosome, and one or a plurality of DNA insertion sites has been transferred, so as to introduce the vector for introducing an exogenous DNA in a site-directed manner into the DNA insertion site of the mammalian artificial chromosome.
(34) The method for preparing a mammalian artificial chromosome vector or a fragment thereof according to (33), wherein the vector for introducing an exogenous DNA further comprises another DNA insertion site in addition to one or a plurality of the DNA insertion sites.

The present invention can provide a Chinese hamster artificial chromosome (CHAC) or a fragment thereof that can introduce a foreign DNA encoding a target useful protein or the like in a site-directed manner into one or a plurality of DNA insertion sites.

Further, the present invention provides a CHAC vector or a fragment thereof comprising a foreign DNA encoding a target useful protein or the like introduced into CHAC. Further, the present invention provides a mammalian cell comprising the CHAC vector or a fragment thereof introduced thereinto. Thus, the present invention can be useful for stable production of biopharmaceuticals, such as human antibodies.

The description of the present application includes the contents disclosed in Japanese Patent Application No. 2023-029116 from which the present application claims priority.

### Brief Description of the Drawings

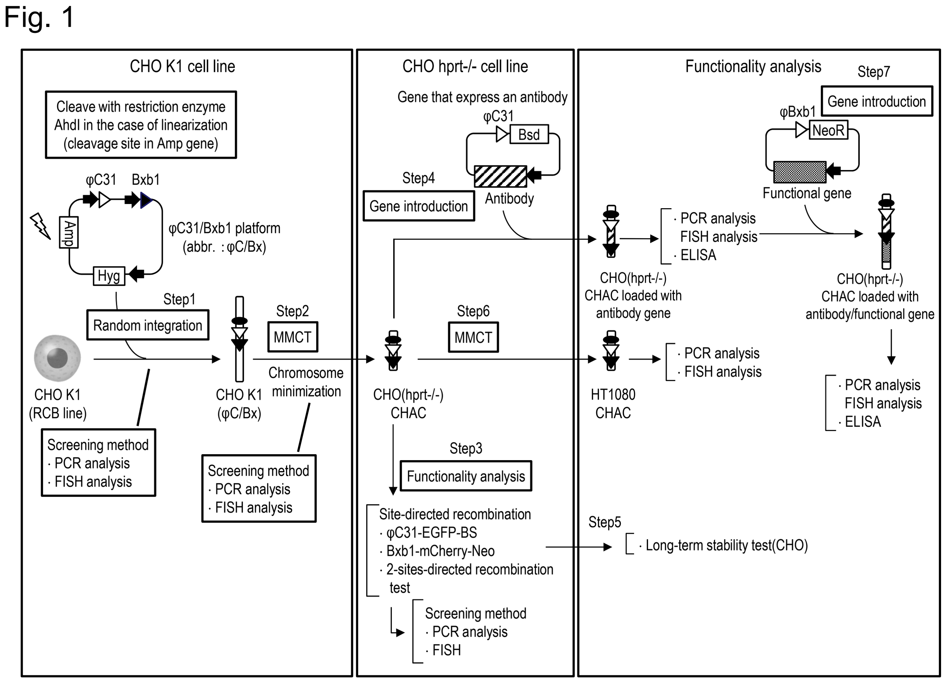

[Figure 1] Figure 1 shows an overall view for preparation of the CHAC vector of the present invention.

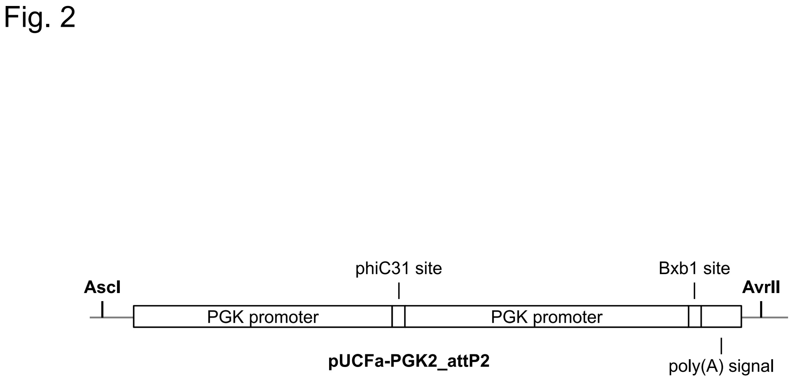

[Figure 2] Figure 2 shows a structure of the PGK-attP synthetic gene comprising bacteriophage phiC31 and Bxb1 attP sites tandemly arranged downstream of the mouse PGK promoter.

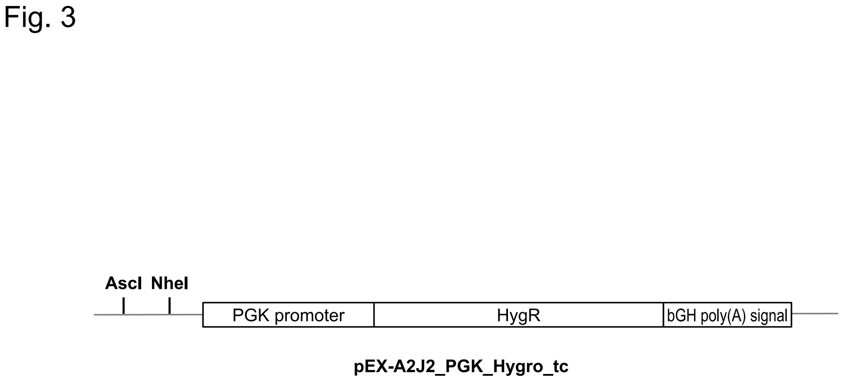

[Figure 3] Figure 3 shows a structure of the PGK-Hygro synthetic gene comprising the hygromycin-resistant gene (HygR) connected to a site downstream of the PGK promoter.

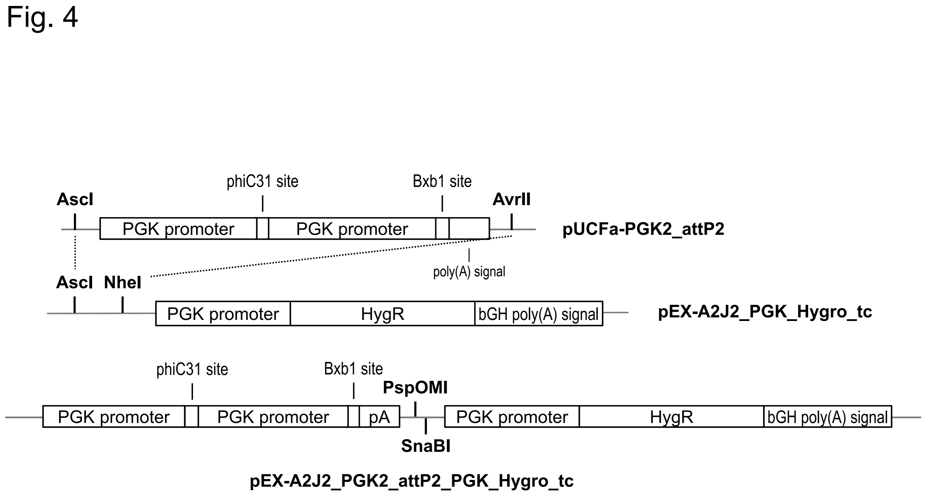

[Figure 4] Figure 4 shows a synthetic gene comprising the PGK-attP synthetic gene ligated to the PGK-Hygro synthetic gene.

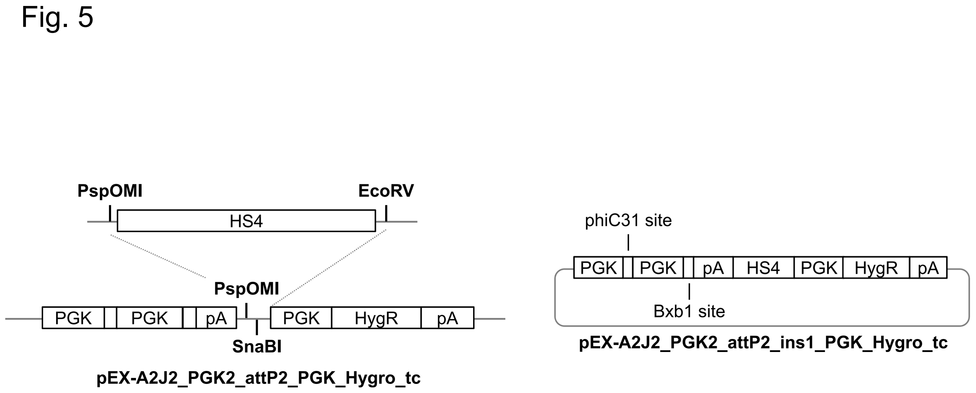

[Figure 5] Figure 5 shows a synthetic gene comprising the chicken β-globin insulator fragment inserted into the PspOMI/SnaBI site of the synthetic gene shown in Figure 4.

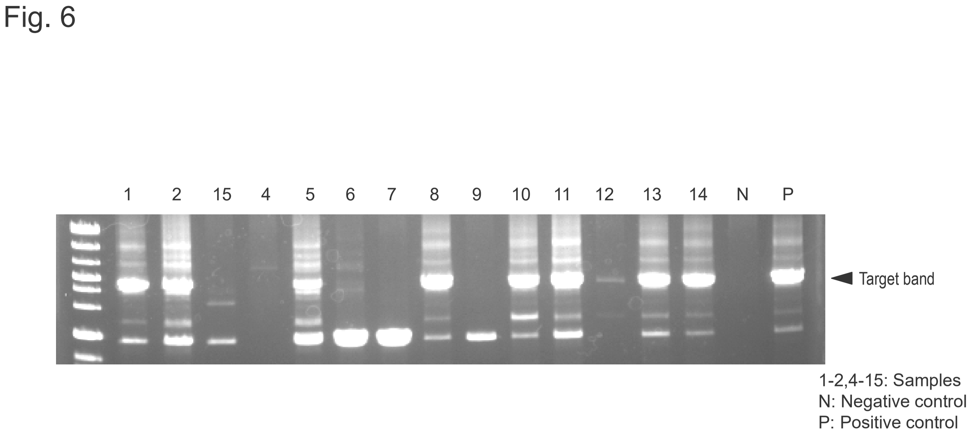

[Figure 6] Figure 6 shows a photograph showing the full-length of a vector for loading an acceptor site detected by PCR so as to confirm the loaded clones. Lanes 1-2 and 4-15 indicate samples, N indicates a negative control, and P indicates a positive control. An arrow indicates a target band.

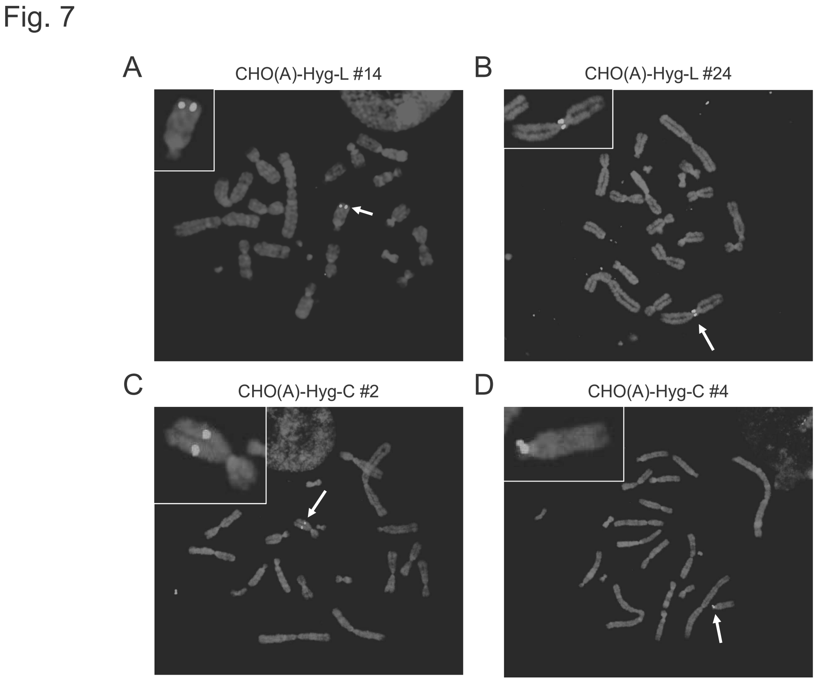

[Figure 7] Figure 7 shows photographs confirming introduction of the acceptor site into CHO-K1 (RCB strain) detected by FISH analysis. Figure 7A shows a photograph of CHO(A)-Hyg-L #14, Figure 7B shows a photograph of CHO(A)-Hyg-L #24, Figure 7C shows a photograph of CHO(A)-Hyg-C #2, and Figure 7D shows a photograph of CHO(A)-Hyg-C #4. A CHO chromosome loaded with the acceptor site introduced thereinto is indicated by an arrow.
Figure 7A to Figure 7D each show an enlarged photograph of the CHO chromosome at the left top corner.

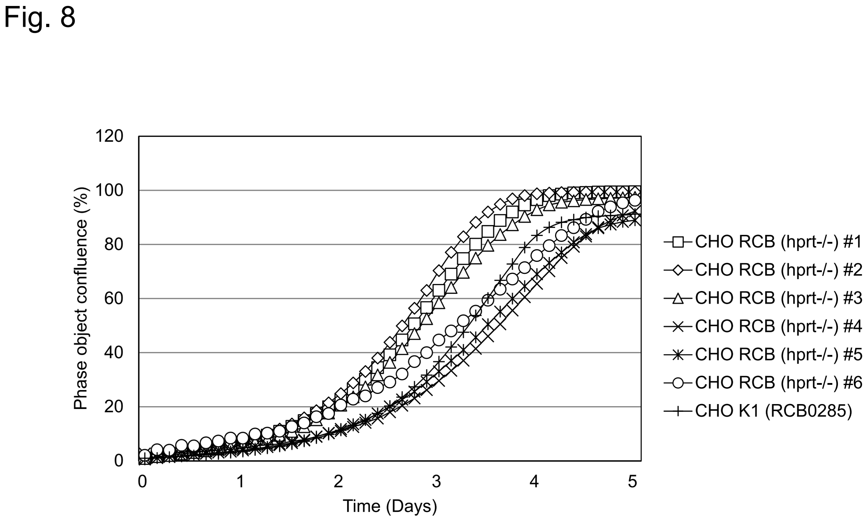

[Figure 8] Figure 8 is a graph demonstrating establishment of HPRT(-)CHO-K1 by the growth of various cell strains. The horizontal axis indicates the time and the vertical axis indicates the phase object confluence (%).

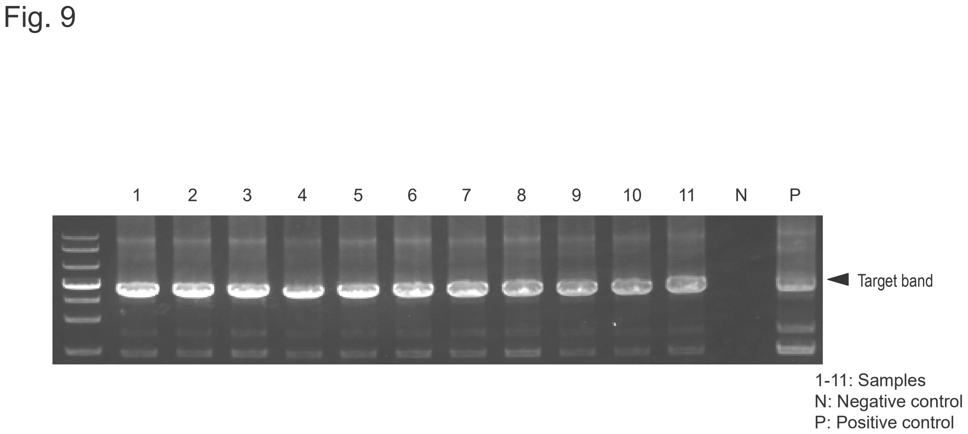

[Figure 9] Figure 9 shows a photograph confirming transfer of the CHO chromosome loaded with the acceptor site introduced into each cell clone by PCR. In Figure 9, the full length of the vector for loading an acceptor site is detected. Lanes 1-11 indicate samples, N indicates a negative control, and P indicates a positive control. An arrow indicates a target band.

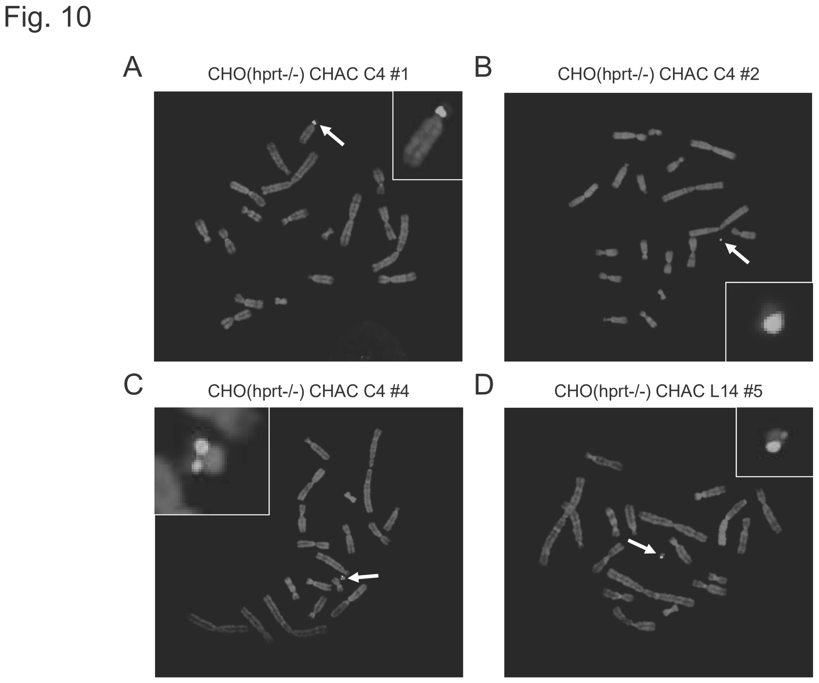

[Figure 10] Figure 10 shows photographs confirming chromosome transfer by the microcell-mediated chromosome transfer (MMCT) method by FISH analysis. Figure 10A shows a photograph of CHO(hppt-/-)CHAC C4 #1, Figure 10B shows a photograph of CHO(hppt-/-)CHAC C4 #2, Figure 10C shows a photograph of CHO(hppt-/-)CHAC C4 #4, and Figure 10D shows a photograph of CHO(hppt-/-)CHAC L14 #5. The detected Chinese hamster artificial chromosome (CHAC) is indicated by an arrow. Figure 10A shows an enlarged photograph of CHAC at the right top corner, Figure 10B shows that at the right bottom corner, Figure 10C shows that at the left top corner, and Figure 10D shows that at the right top corner.

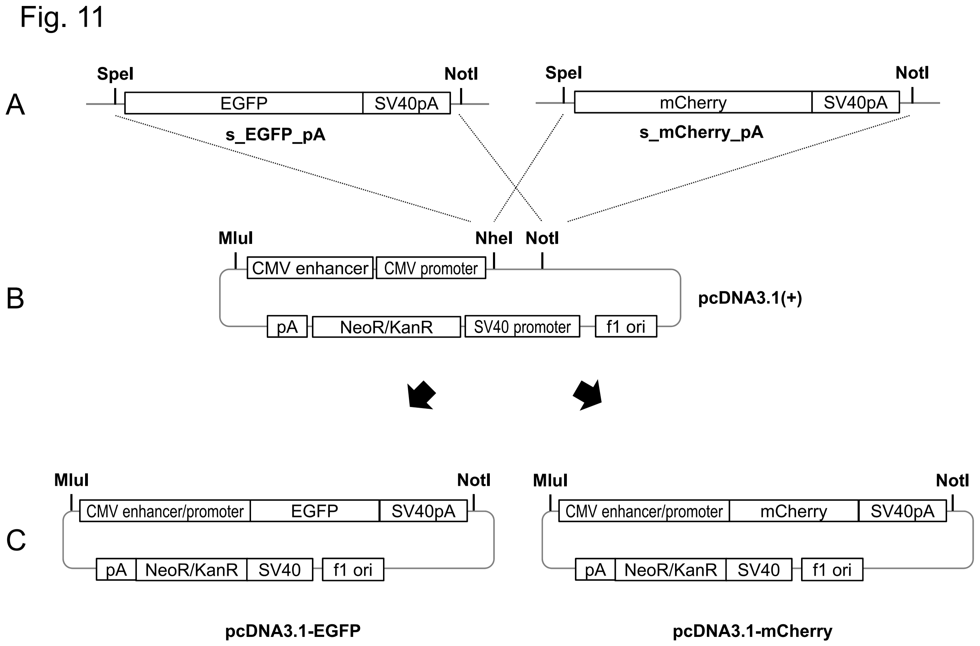

[Figure 11] Figure 11 shows synthesis of the fluorescent protein gene and loading it on an expression vector. Figure 11A shows a structure of the artificially synthesized green fluorescent protein gene EGFP and that of the artificially synthesized red fluorescent protein gene mCherry. Figure 11B shows insertion of such genes into the NheI/NotI site of pcDNA3.1 (Invitrogen). Figure 11C shows a structure of the green fluorescent protein gene loaded on an expression vector (cDNA3.1-EGFP: left) and a structure of the red fluorescent protein gene mCherry loaded on an expression vector (pcDNA3.1-mCherry: right).

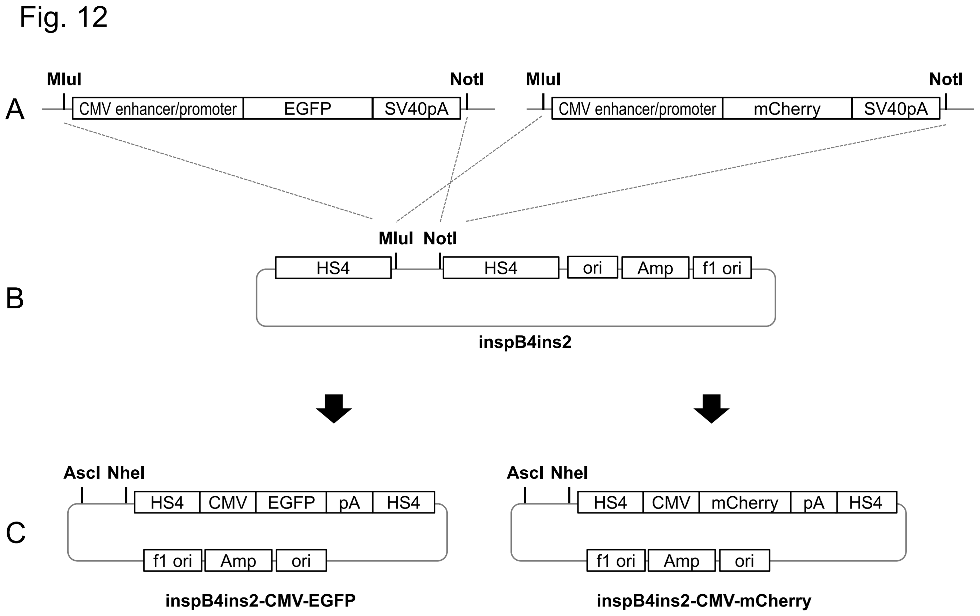

[Figure 12] Figure 12 shows loading of the fluorescent protein gene into an insulator vector. Figure 12A shows a structure of the artificially synthesized green fluorescent protein gene EGFP and that of the artificially synthesized red fluorescent protein gene mCherry. Figure 12B shows insertion of such genes into the MluI/NotI site of the insulator vector. Figure 12C shows a structure of the green fluorescent protein gene EGFP introduced into the MluI/NotI site of an insulator vector (left) and a structure of the red fluorescent protein gene mCherry loaded on the MluI/NotI site of an insulator vector (right).

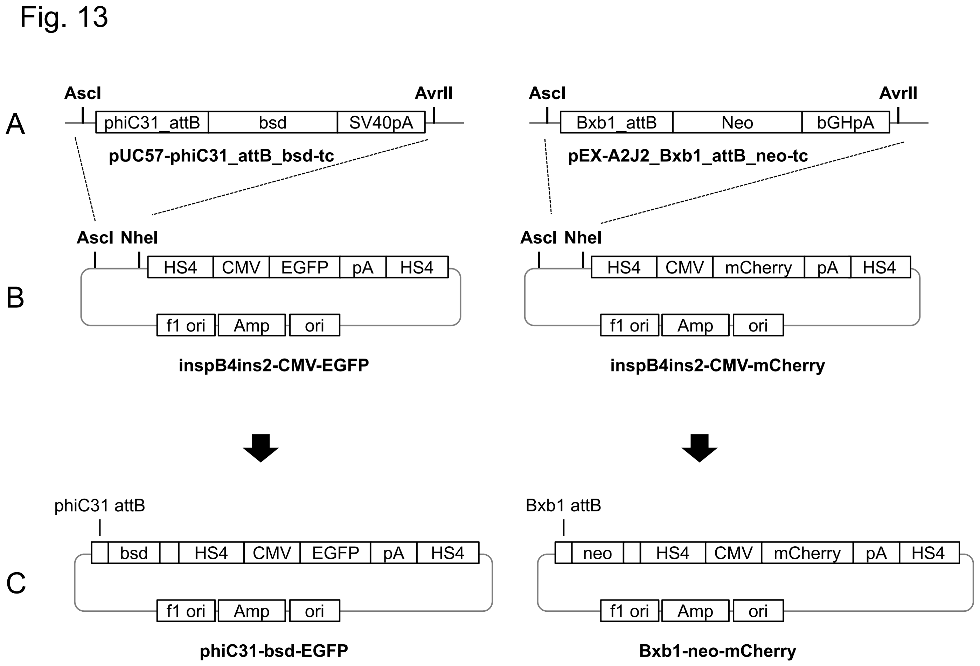

[Figure 13] Figure 13 shows construction of vectors for introducing a fluorescent protein gene into cells (phiC31-bsd-EGFP and Bxb1-neo-mCherry). Figure 13A shows structures of integrase-mediated gene recombination cassettes; i.e., the phiC31-bsd cassette and the Bxb1-neo cassette. As shown in Figure 13B, the cassette shown in Figure 13A is integrated into the AscI/NheI site of the vector shown in Figure 12C. Figure 13C shows structures of vectors for introducing a fluorescent gene into cells mediated by an integrase (phiC31-bsd-EGFP (left) and Bxb1-neo-mCherry (right)).

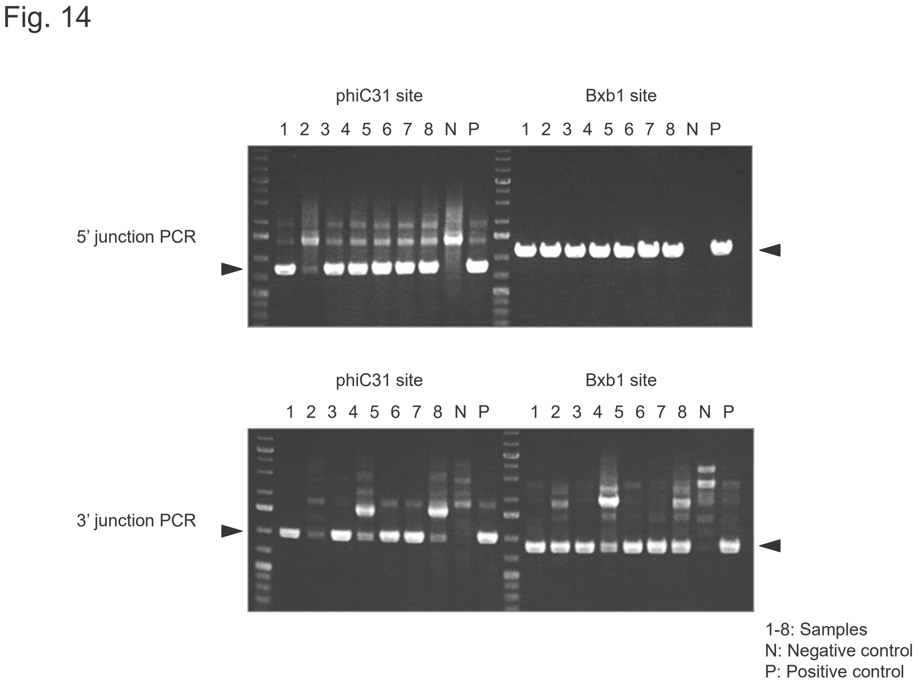

[Figure 14] Figure 14 shows photographs demonstrating the results of validation of the CHAC introduction site by PCR. In Figure 14, the left lanes show detection introduction of the phiC31 site and the right lanes show detection of introduction of the Bxb1 site. In Figure 14, the upper diagram shows the results of 5' junction PCR and the lower diagram shows the results of 3' junction PCR. The left arrow indicates a band of the phiC31 site and the right arrow indicates a band of the Bxb1 site. For each site, lanes 1-8 indicate samples, N indicates a negative control, and P indicates a positive control.

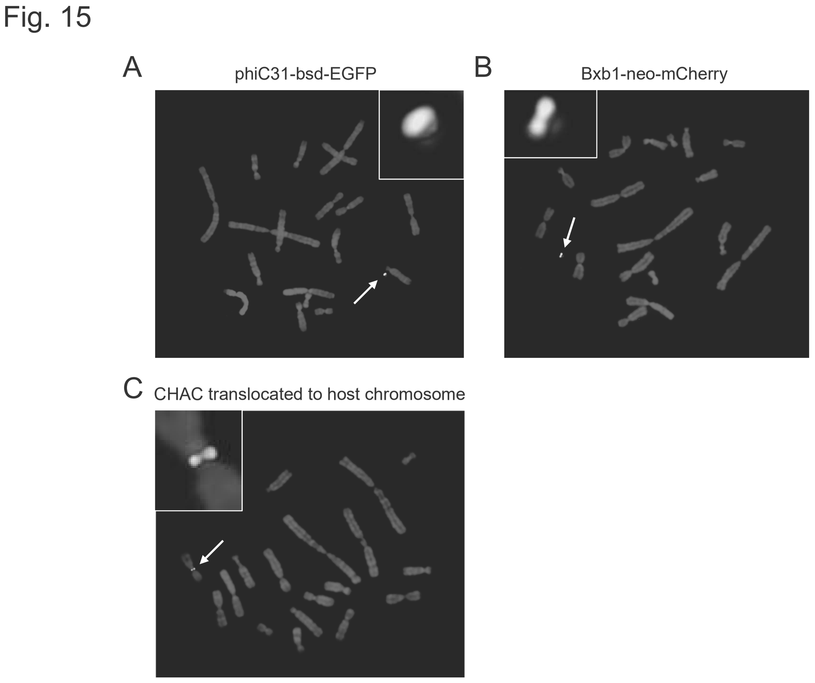

[Figure 15] Figure 15 shows photographs demonstrating the results of validation of the acceptor site by FISH analysis. Figure 15A shows the results on phiC31-bsd-EGFP and Figure 15B shows the results on Bxb1-neo-mCherry. Figure 15C shows the results attained as a result of translocation of CHAC to the host chromosome. The detected Chinese hamster artificial chromosome (CHAC) is indicated by an arrow. Figure 15A shows an enlarged photograph of CHAC at the right top corner, and Figure 15B and Figure 15C each show an enlarged photograph of CHAC at the left top corner.

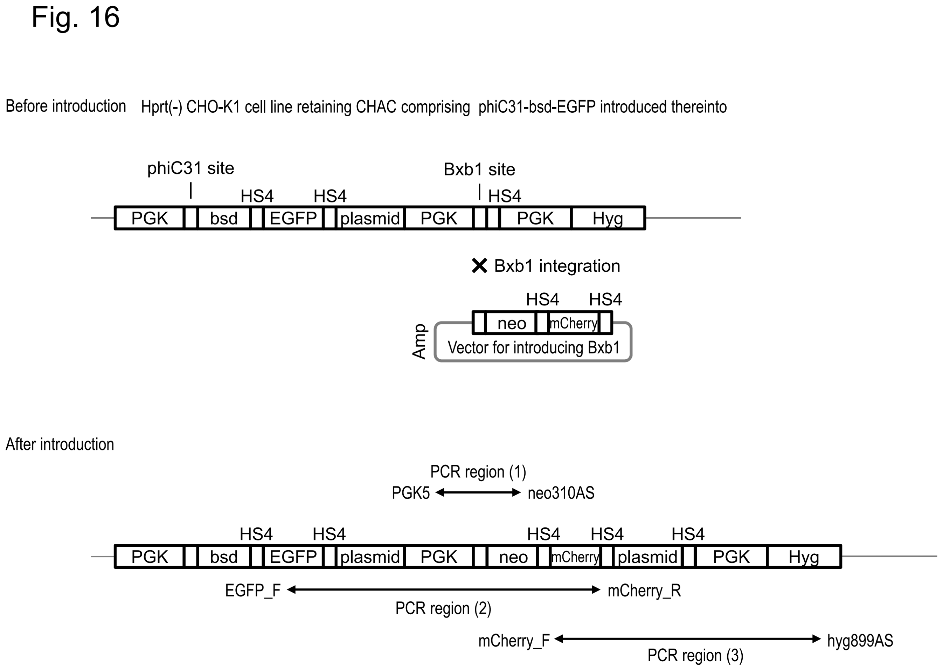

[Figure 16] Figure 16 schematically shows a structure of a vector for loading two-site directed recombination sites. In Figure 16, the upper diagram shows a structure of the vector of CHO (hppt-/-)CHO-K1 line retaining CHAC loaded with phiC31-bsd-EGFP (before loading the vector for loading Bax1), and a structure of the vector for loading Bxb1. The lower diagram shows a structure of the vector after loading, the vector for loading Bxb1.

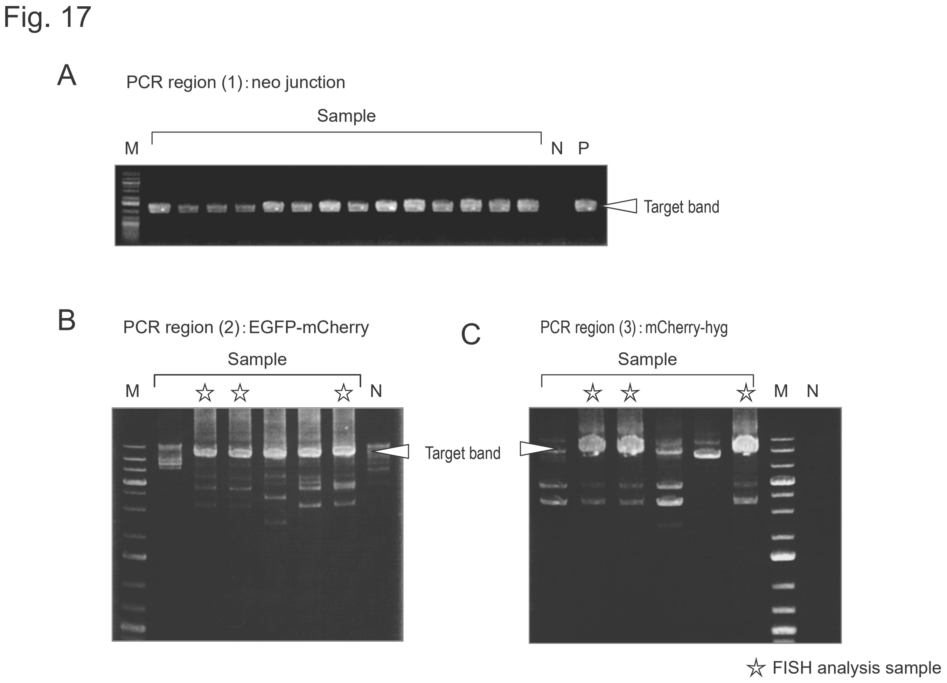

[Figure 17] Figure 17 shows photographs confirming loading of the two-site-directed recombination sites detected by PCR. Figure 17A shows a photograph indicating detection of the neo junction (the PCR region (1)), Figure 17B shows a photograph indicating detection of EGFP-mCherry (the PCR region (2)), and Figure 17C shows a photograph indicating detection of mCherry-hyg (the PCR region (3)). In Figure 17, an arrow indicates a target band, and a star symbol indicates a sample subjected to FISH analysis.

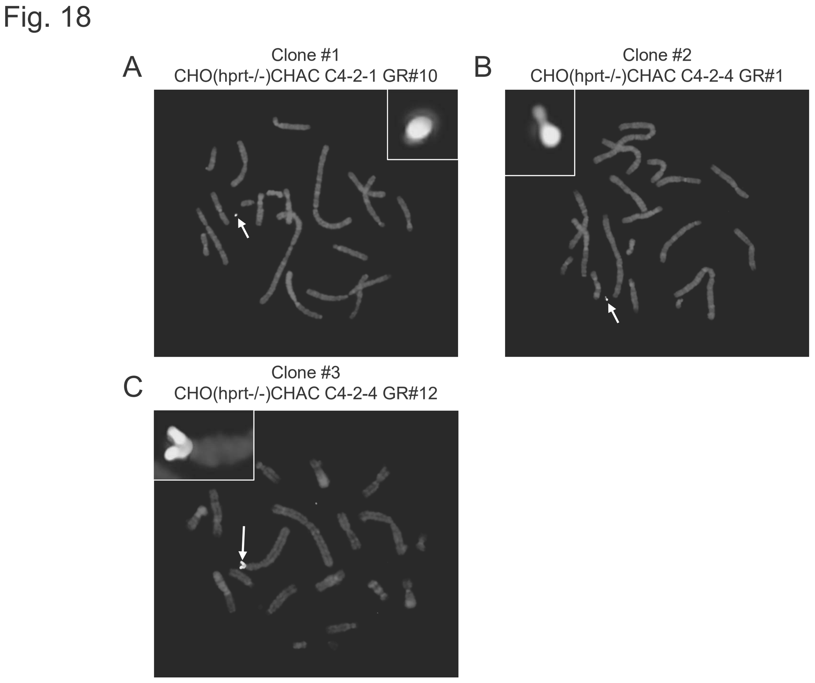

[Figure 18] Figure 18 shows photographs demonstrating the results of verification of the second acceptor site performed by FISH analysis. Figure 18A shows a photograph of Clone #1 (CHO(hprt-/-)CHAC C4-2-1 GR#10), Figure 18B shows a photograph of Clone #2 (CHO(hprt-/-)CHAC C4-2-4 GR#1), and Figure 18C shows a photograph of Clone #3 (CHO(hprt-/-)CHAC C4-2-4 GR#12). The detected CHAC is indicated by an arrow. Figure 18A shows an enlarged photograph of CHAC at the right top corner, and Figure 18B and Figure 18C each show an enlarged photograph of CHAC at the left top corner.

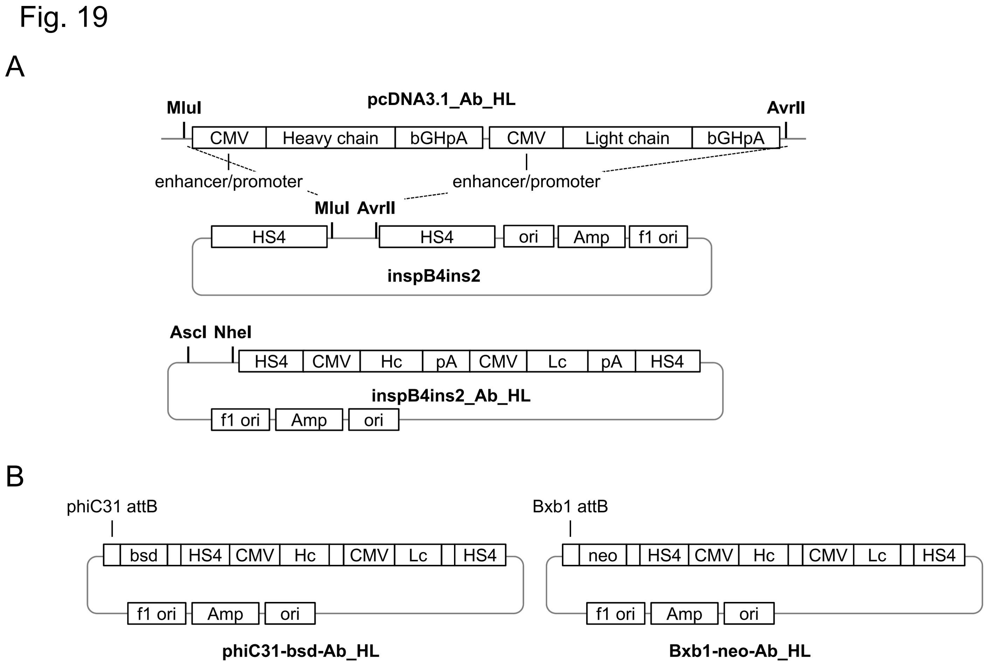

[Figure 19] Figure 19 schematically shows a structure of a vector for AMIGO2 antibody expression. The pcDNA3.1_Ab_HL vector having the full-length heavy chain and light chain regions of the AMIGO2 antibody gene was digested with MluI-AvrII and inserted into the MluI-AvrII site of inspB4ins2 (the upper diagram in Figure 19A) to prepare inspB4ins2_Ab_HL (the lower diagram in Figure 19A). The phiC31-bsd cassette and the Bxb1-neo cassette (see Figure 13A) were inserted into the AscI-NheI site of inspB4ins2_Ab_HL to construct phiC31-bsd-Ab_HL and Bxb1-neo-Ab_HL (Figure 19B).

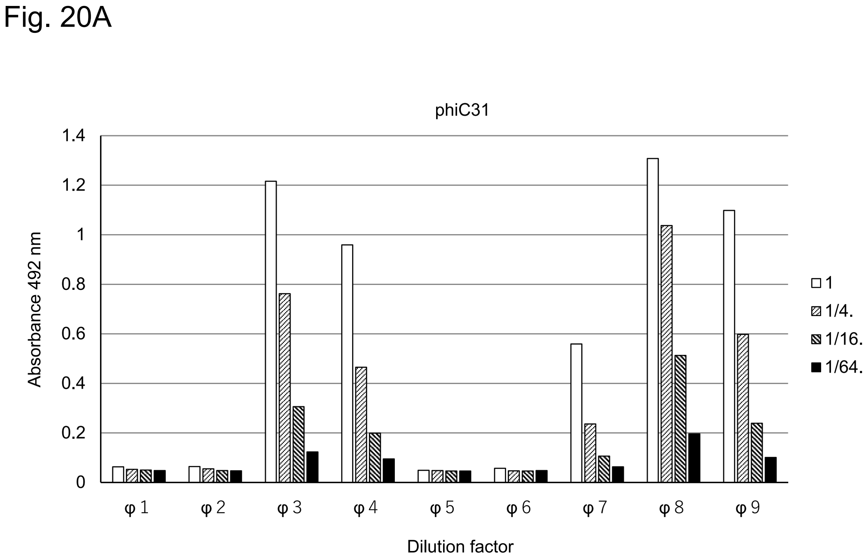

[Figure 20A] Figure 20A shows a chart demonstrating the AMIGO2 antibody productivity from clones comprising the genes introduced into the φC31 site analyzed by ELISA. The horizontal axis indicates a dilution factor and the vertical axis indicates the absorbance at 492 nm.

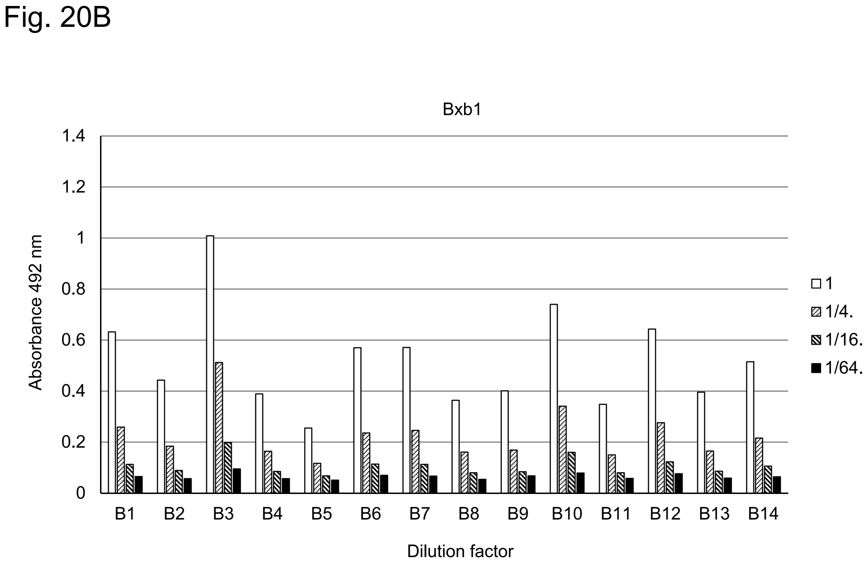

[Figure 20B] Figure 20B shows a graph demonstrating the AMIGO2 antibody productivity from clones comprising the genes introduced into the Bxb1 site analyzed by ELISA. The horizontal axis indicates a dilution factor and the vertical axis indicates the absorbance at 492 nm.

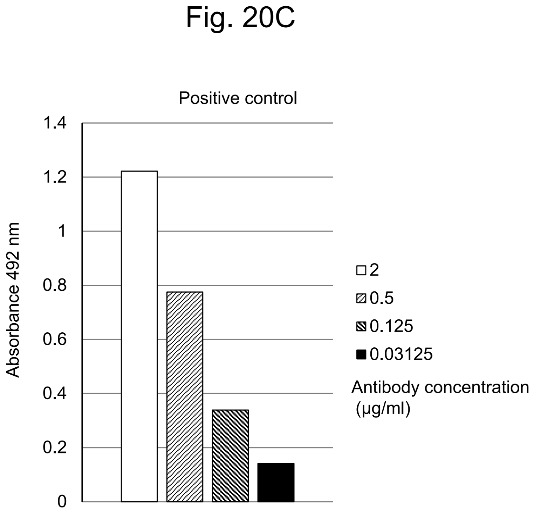

[Figure 20C] Figure 20C shows a chart showing the results of ELISA analysis performed by adjusting the purified antibody concentration using the positive control antibody. The horizontal axis indicates the purified antibody concentration and the vertical axis indicates the absorbance at 492 nm.

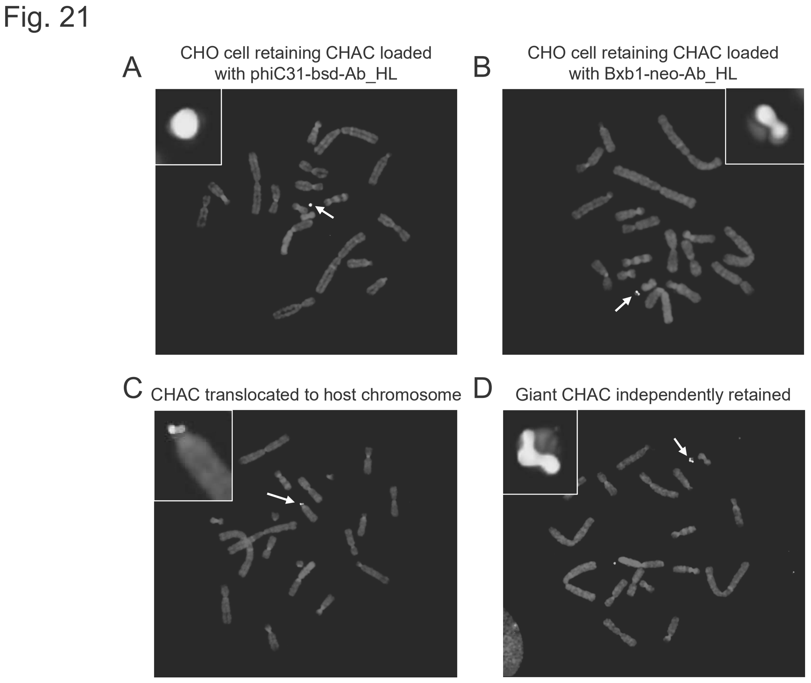

[Figure 21] Figure 21 shows photographs confirming loading of the antibody gene into CHAC detected by FISH analysis. Figure 21A shows the results on CHO cells retaining CHAC loaded with phiC31-bsd-Ab_HL introduced thereinto, Figure 21B shows the results on CHO cells loaded with CHAC comprising Bxb1-neo-Ab_HL introduced thereinto. Figure 21C shows a photograph demonstrating translocation of CHAC to the host chromosome and Figure 21D shows a photograph showing independently maintained CHAC that has become giant as a result of structural changes. The detected CHAC is indicated by an arrow. Figure 21A shows an enlarged photograph of CHAC at the left top corner and Figure 21B, Figure 21C, and Figure 21D each show an enlarged photograph of CHAC at the right top corner.

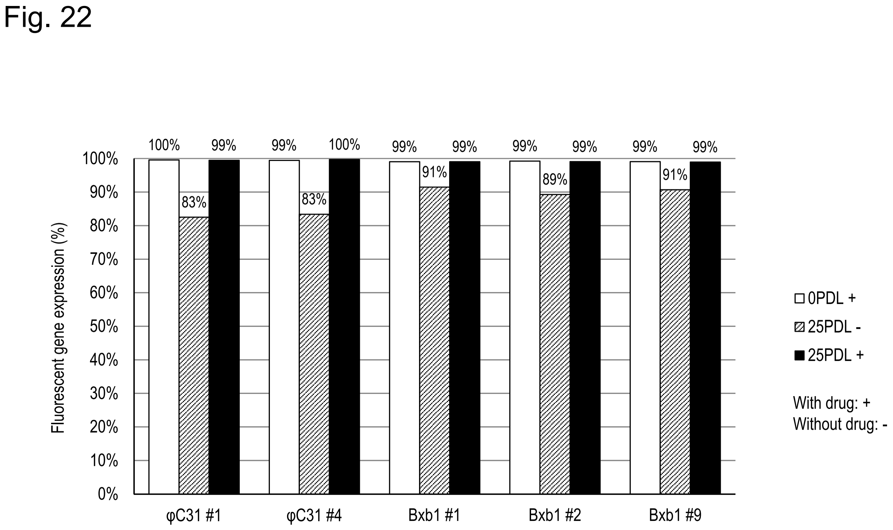

[Figure 22] Figure 22 shows a graph demonstrating the results of examination of long-term stability of CHAC loaded with the fluorescent gene introduced thereinto performed by ECM analysis. The chart shows, from the left to the right, the results on the cells loaded with the φC31#1 clone, the φC31#4 clone, the Bxb1#1 clone, the Bxb1#2 clone, and the Bxb1#9 clone introduced thereinto. The vertical axis indicates the rate of fluorescent gene-expressing cells (%). Columns for each clone show, from the left to the right, the results attained at 0PDL+, 25PDL-, and 25PDL+.

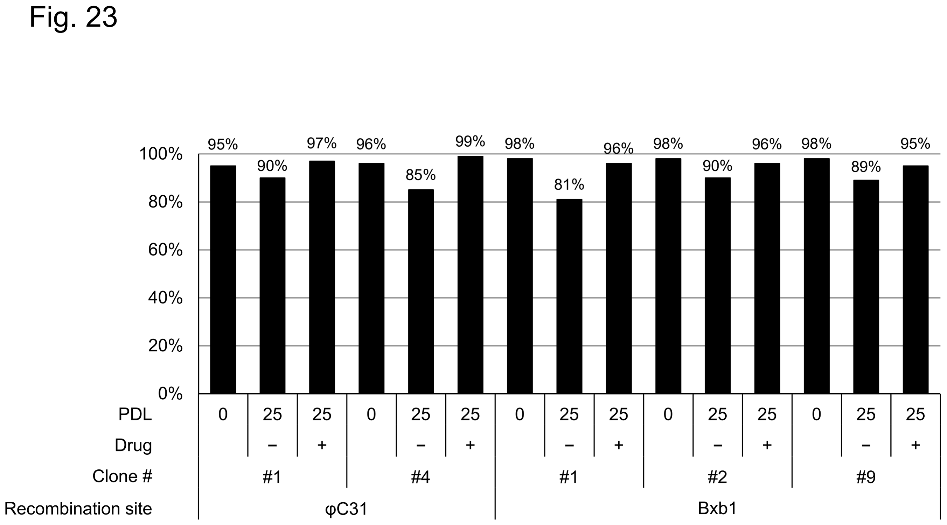

[Figure 23] Figure 23 shows a graph demonstrating the results of examination of long-term stability of CHAC performed by FISH analysis. The chart shows, from the left to the right, the results on the cells loaded with the φC31#1 clone, the φC31#4 clone, the Bxb1#1 clone, the Bxb1#2 clone, and the Bxb1#9 clone introduced thereinto. The vertical axis indicates the CHAC-retaining rate (%). Columns for each clone show, from the left to the right, the results attained at 0PDL+, 25PDL-, and 25PDL+.

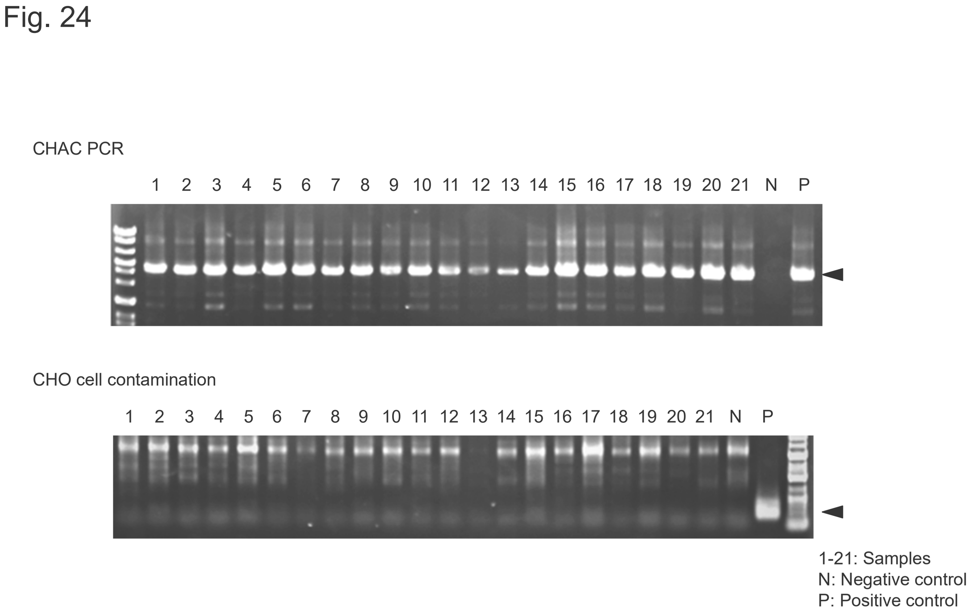

[Figure 24] Figure 24 shows photographs demonstrating CHAC transfer into the HT1080 cells detected by PCR. The upper photograph shows PCR of CHAC and the lower photograph demonstrating that CHO cells are not contaminated.

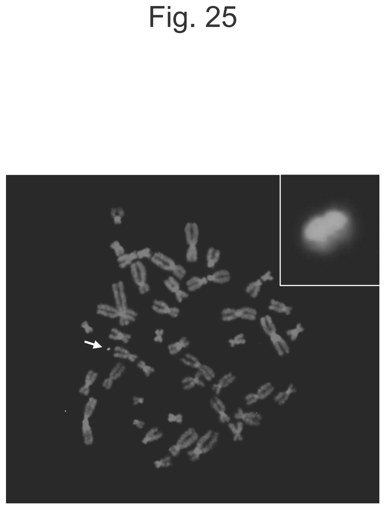

[Figure 25] Figure 25 shows a photograph confirming CHAC transfer into HT1080 cells by the MMCT method detected by FISH analysis. The detected CHAC is indicated by an arrow. Figure 25 shows an enlarged photograph of CHAC at the right top corner.

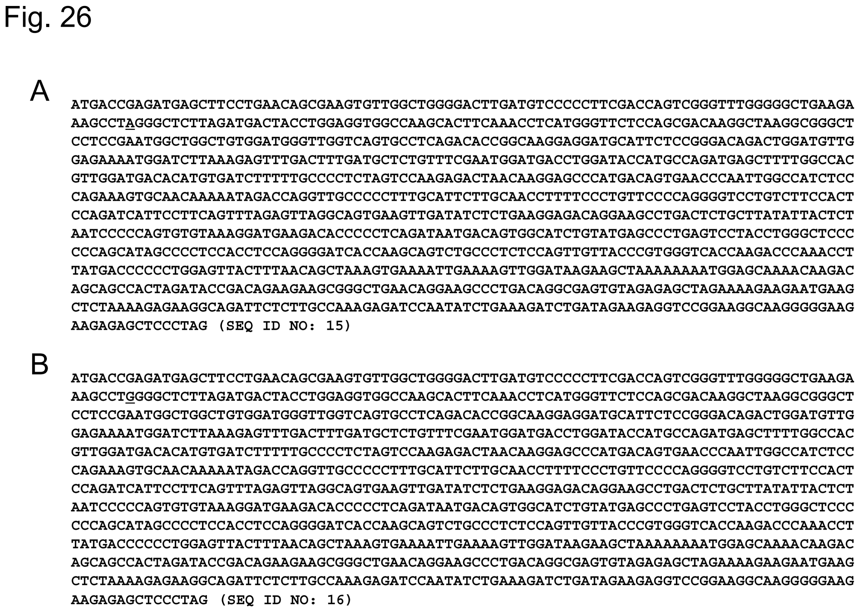

[Figure 26] Figure 26 shows the sequence information of the gene encoding ATF4. Figure 26A shows the nucleotide sequence under Accession Number: XM_007654285. Figure 26B shows the information of the modified and synthesized sequence.

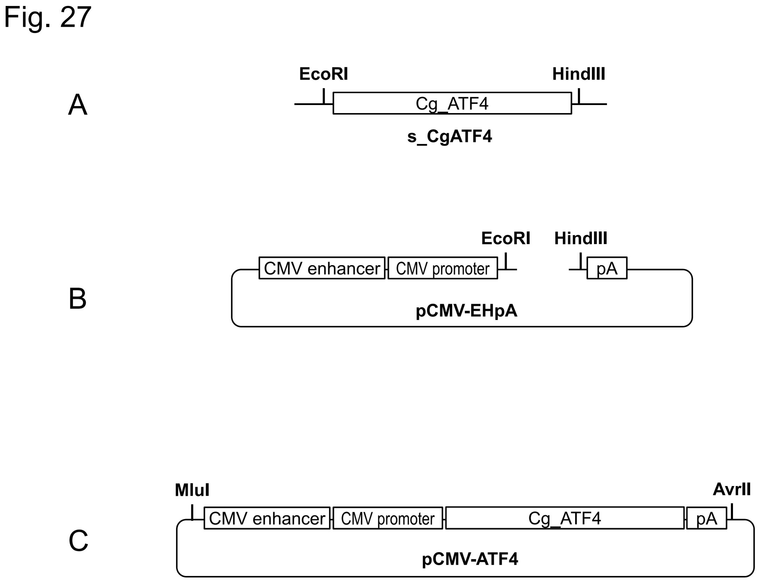

[Figure 27] Figure 27 shows a structure of the vector for loading ATF4. Figure 27A shows a structure of synthesized s_CgATF4. Figure 27B shows a structure of the pCMV-EHpA expression vector for inserting s_CgATF4. Figure 27C shows a structure of the pCMV-ATF4 vector comprising s_CgATF4 inserted into pCMV-EHpA.

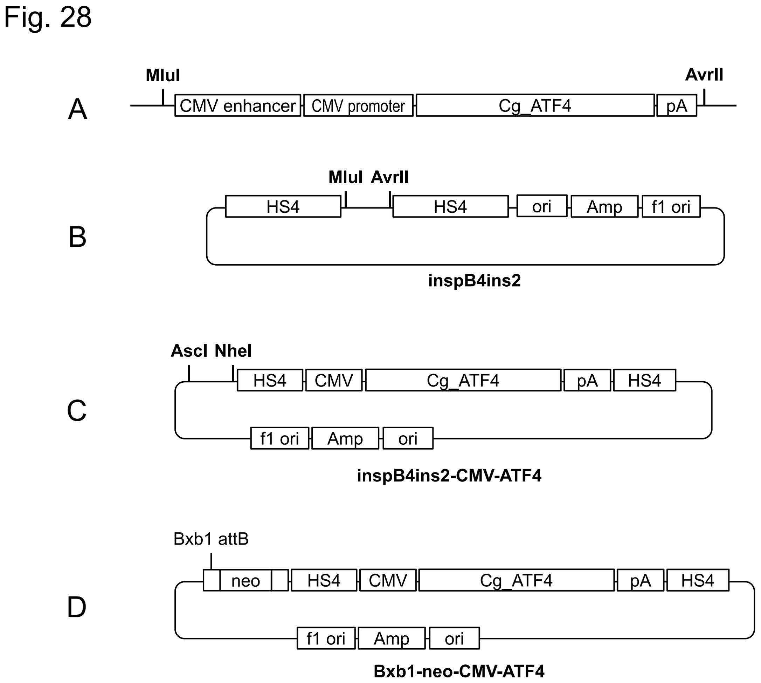

[Figure 28] Figure 28 demonstrates the ATF4 expression unit that is modified to enable loading on CHAC. Figure 28A shows a structure of the ATF4 expression unit, Figure 28B shows a structure of the inspB4ins2 vector for inserting the expression unit, and Figure 28C shows a structure of inspB4ins2-CMV-ATF4 comprising the ATF4 expression unit inserted into the inspB4ins2 vector. Figure 28D shows a structure of Bxb1-neo-CMV-ATF4 in which Bxb1-attB-neo inserted into inspB4ins2-CMV-ATF4.

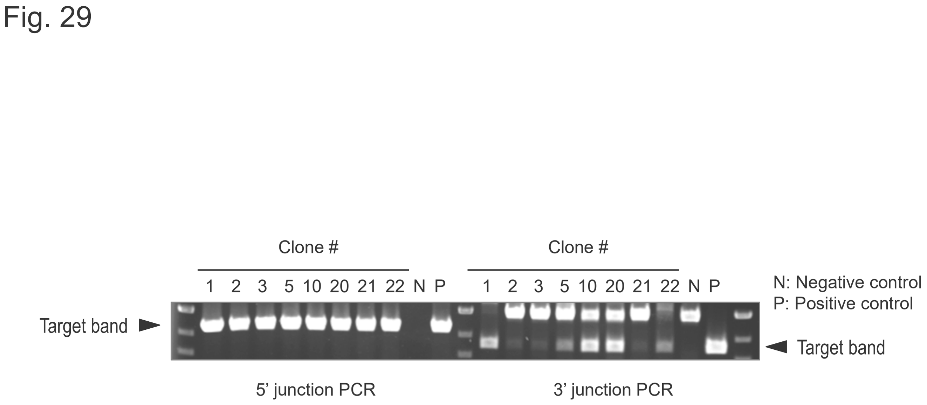

[Figure 29] Figure 29 shows the results of PCR analysis of the clones obtained by loading Bxb1-neo-CMV-ATF4 into CHAC retaining the AMIGO2 antibody gene. Figure 29 shows the results of analysis of the upstream junction (5' junction PCR) on the left and the results of analysis of the downstream junction (3' junction PCR) on the right. An arrow indicates a target band, N indicates a negative control, and P indicates a positive control.

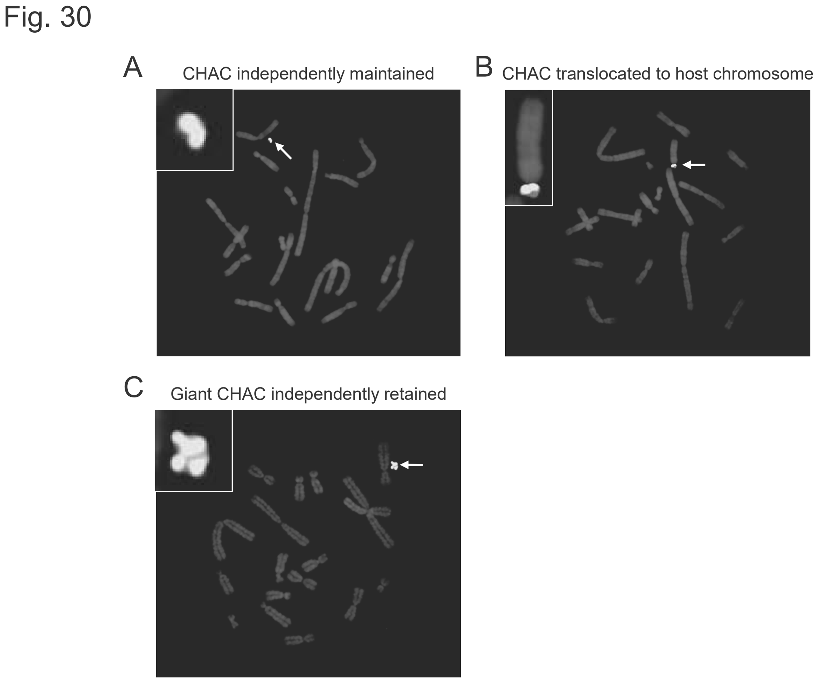

[Figure 30] Figure 30 shows the results of FISH analysis of the clones obtained by loading Bxb1-neo-CMV-ATF4 into CHAC retaining the AMIGO2 antibody gene. Figure 30A shows a photograph of independently maintained CHAC, Figure 30B shows a photograph of CHAC translocated to the host chromosome, and Figure 30C shows a photograph of independently maintained CHAC that has become giant as a result of structural changes. Figure 30A, Figure 30B, and Figure 30C each show an enlarged photograph of CHAC at the left top corner.

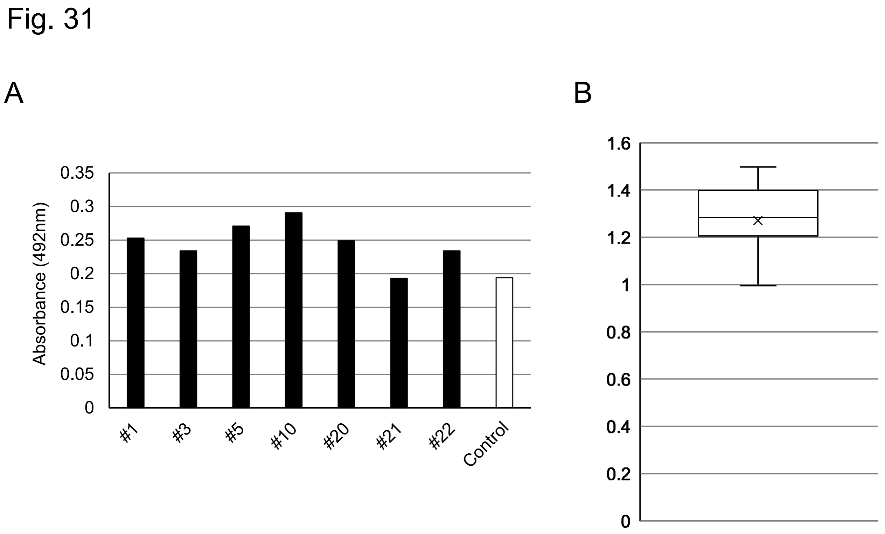

[Figure 31] Figure 31 shows the results of ELISA analysis of the AMIGO2 antibody productivity from the clones obtained by loading Bxb1-neo-CMV-ATF4 into CHAC retaining the AMIGO2 antibody gene. Figure 31A shows a graph demonstrating the results of ELISA analysis for verifying the AMIGO2 antibody productivity from each clone. The vertical axis indicates the absorbance at 492 nm. Figure 31B shows a rate of an improvement in the AMIGO2 antibody productivity achieved by loading ATF4 into CHAC retaining the AMIGO2 antibody gene.

### Embodiments of the Invention

The present invention is described in more detail.

### 1. The Chinese hamster artificial chromosome of the present invention or a fragment thereof

The present invention provides a Chinese hamster artificial chromosome or a fragment thereof comprising a centromere and a telomere derived from a Chinese hamster chromosome, a part of the chromosome, and one or a plurality of DNA insertion sites.

The terms "a centromere derived from a Chinese hamster chromosome" and "a telomere derived from a Chinese hamster chromosome" used herein refer to a centromere and a telomere derived from a natural Chinese hamster chromosome, respectively. In the Chinese hamster chromosome of the present invention, specifically, a centromere and a telomere naturally exist in the Chinese hamster chromosome, and neither the centromere nor telomere is artificial.

The term "a fragment of the Chinese hamster artificial chromosome" used herein refers to a fragment that is derived from the Chinese hamster artificial chromosome of the present invention and maintains functions as the Chinese hamster artificial chromosome. A fragment of the Chinese hamster artificial chromosome of the present invention has substantially no endogenous long-arm or short-arm gene of a Chinese hamster and it comprises a small chromosome region, a telomere, and a centromere derived from the Chinese hamster and a DNA insertion site.

The term "part of the chromosome (Chinese hamster chromosome)" used herein refers to a part of a chromosome derived from a Chinese hamster, such as a region proximal to the centromere- or the telomere.

A nucleotide size of the Chinese hamster artificial chromosome of the present invention or a fragment thereof is not particularly limited, provided that the chromosome or a fragment thereof comprises a centromere and a telomere derived from a Chinese hamster chromosome and a part of the chromosome. A nucleotide size thereof is shorter than that of a conventional artificial chromosome. Specifically, the Chinese hamster artificial chromosome of the present invention or a fragment thereof selectively comprises, in addition to the centromere and the telomere derived from a natural Chinese hamster, a minimal part of the chromosome that is necessary to function as the chromosome; i.e., a centromere- and telomere-proximal regions of the chromosome.

A nucleotide size of the Chinese hamster artificial chromosome of the present invention or a fragment thereof is 30 Mbp or less, 20 Mbp or less, 10 Mbp or less, 5 Mbp or less, or 3 Mbp or less, and it is particularly preferable that the nucleotide size be 3 Mbp or less. A short nucleotide size of the Chinese hamster artificial chromosome or a fragment thereof indicates that a region derived from the Chinese hamster chromosome other than the centromere and the telomere is minimized.

In the Chinese hamster chromosome of the present invention or a fragment thereof, it is particularly preferable that all the sequence other than the DNA insertion site be derived from the Chinese hamster chromosome. This can avoid disadvantages, such as generation of undesirable products caused by inclusion of chromosome components derived from other organism species.

The term "DNA insertion site" used herein refers to a site of an artificial chromosome into which a target exogenous DNA (e.g., a gene or gene locus) can be inserted, such as a site-directed recombinase recognition site. A DNA insertion site to be introduced into the Chinese hamster chromosome of the present invention or a fragment thereof is not particularly limited, provided that an exogenous DNA can be inserted into the site. Examples of recognition sites include Bxb1 attP and Bxb1 attB sequences, φC31 attB and φC31 attP sequences, R4 attB and R4 attP sequences, TP901-1 attB and TP901-1 attP sequences, an FRT sequence, and a loxP sequence. While the detail is described below, in the present invention, such DNA sequence insertion sites can be introduced by random integration instead of site-directed introduction.

In the present invention, the Chinese hamster artificial chromosome or a fragment thereof comprises one recognition site or a plurality of recognition sites. Specifically, the Chinese hamster artificial chromosome or a fragment thereof can comprise a plurality of recognition sites of different types. It is possible to introduce an exogenous DNA in a site-directed manner into each of a plurality of recognition sites. An exogenous DNA to be introduced into the DNA insertion site of the Chinese hamster chromosome is described in detail in 2. below.

The Chinese hamster artificial chromosome of the present invention or a fragment thereof can comprise either or both of a reporter gene and a selection marker gene. The Chinese hamster artificial chromosome of the present invention or a fragment thereof comprises a reporter gene, such as a fluorescent gene. This enables detection of the Chinese hamster chromosome using the fluorescence as the indicator. Inclusion of a selection marker gene, such as an antibiotic-resistant gene, enables selection of a cell comprising the Chinese hamster chromosome by screening.

Examples of reporter genes include, but are not particularly limited to, a fluorescent protein gene (e.g., a green fluorescent protein (GFP or EGFP) gene or a yellow fluorescent protein (YFP) gene), a tag-protein-encoding DNA, a β-galactosidase gene, and a luminescent gene (e.g., a luciferase gene), with GFP or EGFP being preferable.

As a selection marker gene, for example, either or both of a positive selection marker gene and a negative selection marker gene may be used. Examples of positive selection marker genes include drug-resistant genes such as a neomycin-resistant gene (Neo or NeoR), an ampicillin-resistant gene, a hygromycin-resistant gene (Hyg), a blasticidin S (BS)-resistant gene, a puromycin-resistant gene (Puro), a geneticin (G418)-resistant gene, and a zeocin-resistant gene. Examples of negative selection marker genes include a herpes simplex thymidine kinase (HSV-TK) gene, a diphtheria toxin A fragment (DT-A) gene, and a cytosine deaminase uracil phosphoribosyltransferase fusion gene (Fcy::Fur). In general, HSV-TK is used in combination with ganciclovir or cyclovir. Fcy::Fur is used in combination with 5-fluorocytosine (5-FC).

### 2. The Chinese hamster artificial chromosome vector of the present invention or a fragment thereof

The present invention relates to a Chinese hamster artificial chromosome vector or a fragment thereof comprising an exogenous DNA introduced in a site-directed manner into the DNA insertion site of the Chinese hamster artificial chromosome or a fragment thereof described in 1. above. A Chinese hamster artificial chromosome or a fragment thereof comprising an exogenous DNA introduced thereinto functions as a type of a vector that introduces the exogenous DNA into a mammalian cell. Accordingly, a Chinese hamster artificial chromosome or a fragment thereof comprising an exogenous DNA is referred to as a "Chinese hamster artificial chromosome vector or a fragment thereof" herein.

The Chinese hamster artificial chromosome or a fragment thereof comprises a site into which an exogenous DNA is to be introduced (i.e., a DNA insertion site). The Chinese hamster artificial chromosome vector of the present invention or a fragment thereof is prepared by introducing a target exogenous DNA into the site. In order to introduce an exogenous DNA, a vector comprising an exogenous DNA (hereafter, such vector is referred to as a "vector for introducing an exogenous DNA") can be used. A vector for introducing an exogenous DNA may be transfected, so that the vector for introducing an exogenous DNA can be introduced in a site-directed manner into the DNA insertion site of the mammalian artificial chromosome.

In the examples below, a Chinese hamster artificial chromosome comprising two DNA insertion sites is prepared, and the vector is verified to be able to comprise an exogenous gene introduced into all recognition sites. When such vector is introduced into a mammalian-derived cell, the exogenous DNA can be expressed, and, therefore, the Chinese hamster artificial chromosome vector of the present invention or a fragment thereof can be used for protein production and other applications.

It should be noted that at least one insulator sequence may be present at a proximal region or both sides of the insertion site of an exogenous gene DNA in the artificial chromosome vector or a fragment thereof. The insulator sequence exerts an enhancer blocking effect (i.e., adjacent genes do not affect each other) or a chromosome boundary effect (i.e., a region that guarantees gene expression is separated and distinguished from a region inhibiting the gene expression). Examples of such sequences include human β-globin HS1 to HS5 and chicken β-globin HS4.

The Chinese hamster artificial chromosome vector of the present invention or a fragment thereof comprises a plurality of recognition sites, and it thus can introduce a plurality of exogenous DNAs through such a plurality of recognition sites. Accordingly, the Chinese hamster artificial chromosome vector of the present invention or a fragment thereof may comprise a plurality of exogenous DNAs.

The Chinese hamster artificial chromosome vector of the present invention or a fragment thereof may further comprise another DNA insertion site in addition to one or a plurality of the DNA insertion sites described above. After the introduction of an exogenous DNA through, for example, one or a plurality of DNA insertion sites, specifically, another DNA insertion site can further be added to the Chinese hamster artificial chromosome, according to need. The another DNA insertion site to be introduced herein are as described in 1. above. It is possible to further introduce other exogenous DNAs through the additionally introduced DNA insertion sites.

The term "exogenous DNA" used herein refers to an exogenous DNA to be inserted into a gene insertion site of the Chinese hamster artificial chromosome vector or a fragment thereof: i.e., DNA that does not inherently exist in the target cell and is intended to be expressed in the target cell. In the present invention, an exogenous DNA is not particularly limited, provided that it encodes a useful protein that is intended to be expressed.

A nucleotide size of an exogenous DNA is not particularly limited. It may be 20 kb or less or exceed 20 kb, such as 50 kb or more, 100 kb or more, 200 kb or more, 500 kb or more, 700 kb or more, 1 Mb or more, 10 Mb or more, 20 Mb or more, 30 Mb or more, 40 Mb or more, or 50 Mb or more. The Chinese hamster artificial chromosome vector of the present invention or a fragment thereof can load an exogenous DNA (a chromosome fragment) of 1 Mb or more, which is difficult to be carried by artificial chromosome vectors such as BAC, PAC, and YAC vectors.

In the present invention, an exogenous DNA is preferably a human DNA, and more preferably a DNA of a human gene or human chromosome locus. It is further preferable that the exogenous DNA of the present invention be a gene encoding an immunoglobulin heavy chain and/or light chain or DNA of such gene.

Specifically, such exogenous DNA may be a gene encoding an antibody that targets AMIGO2 expressed on the surface of cancer cells. In the examples below, a Chinese hamster artificial chromosome vector is prepared by introducing a gene consisting of the nucleotide sequence of the AMIGO2 antibody heavy chain as shown in SEQ ID NO: 13 of the sequence listing and the nucleotide sequence of the AMIGO2 antibody light chain as shown in SEQ ID NO: 14, and production of antibodies is actually confirmed.

Examples of the exogenous DNAs include, but are not limited to, genes encoding polypeptides, such as cytokines, hormones, growth factors, neurotrophic factors, hematopoietic factors, coagulation or hemolysis factors, G protein-coupled receptors, and enzymes; genes used for treatment of various diseases, such as tumor, muscular dystrophy, hemophilia, neurodegenerative diseases (e.g., Alzheimer's disease, Huntington's disease, and Parkinson's disease), autoimmune diseases, allergic diseases, and genetic diseases; and genes selected from the group consisting of genes or DNAs involved in the immune system such as T cell receptors (TCR) and human leukocyte antigens (HLA).

Examples of cytokines include interferons (e.g., IF-α, IF-β, and IF-γ), interleukins (e.g., IL-1, IL-2, IL-4, IL-6, IL-11, and IL-12), tumor necrosis factors (e.g., TNF-α and TNF-β), and TGF-β family proteins (e.g., bone morphogenic protein (BMP)).

Examples of hormones include growth hormones, human chorionic gonadotropin (hCG), human placental lactogen (hPL), human pituitary gonadotropic hormone, thyroid-stimulating hormone (TSH), a luteinizing hormone-releasing factor, insulin, glucagon, somatostatin, and prolactin.

Examples of growth factors or neurotrophic factors include an insulin-like growth factor, a brain-derived neurotrophic factor (BDNF), an albumin-fused ciliary neurotrophic factor, a platelet-derived neurotrophic factor (PDNF), a transforming growth factor, a nerve growth factor (NGF), and a TNF growth factor. Examples of hematopoietic factors include erythropoietin, a (granulocyte) colony-stimulating factor, and thrombopoietin. Examples of the coagulation or hemolysis factors include Factor VII, Factor VIII, Factor X, and t-PA. Examples of G protein-coupled receptors include an adrenergic receptor, a muscarinic acetylcholine receptor, an adenosine receptor, a GABA receptor (type B), an angiotensin receptor, a cholecystokinin receptor, a dopamine receptor, a glucagon receptor, a histamine receptor, an odorant receptor, an opioid receptor, a secretin receptor, a somatostatin receptor, a gastrin receptor, and a P2Y receptor. Examples of enzymes include asparaginase, superoxide dismutase, uricase, streptokinase, dopamine synthase, and adenosine deaminase.

Examples of genes used for treatment of various diseases, such as tumor, muscular dystrophy, hemophilia, neurodegenerative diseases (e.g., Alzheimer's disease, Huntington's disease, and Parkinson's disease), autoimmune diseases, allergic diseases, and genetic diseases include dystrophin gene, IL-12 gene, TNF-α gene, tumor suppressor gene, dopamine synthase gene, and genetically deficient enzyme genes (e.g., adrenal enzyme genes, such as cytochrome enzyme (P450) gene, 3β-hydroxysteroid dehydrogenase gene, and 21 hydroxylase (P450 c21) gene).

Examples of immune system genes or DNAs include genes encoding proteins involved in the immune system, such as T cell receptors (TCRs), human leukocyte antigens (HLAs), Fcy receptors (FCGRs), killer cell Ig-like receptors (KIRs), and leukocyte Ig-like receptors (LILRs).

The Chinese hamster artificial chromosome vector of the present invention or a fragment thereof is transferred into a mammal as described in 3. below and it is stably retained therein over a long period of time. Accordingly, the Chinese hamster artificial chromosome vector of the present invention or a fragment thereof is useful for stable production of a useful protein that is intended to be expressed.

In the present invention, in addition, the Chinese hamster artificial chromosome vector of the present invention or a fragment thereof can further comprise a functional nucleic acid, in addition to an exogenous DNA encoding the useful protein that is intended to be expressed.

The functional nucleic acid used in the present invention may be a gene that enhances productivity of a protein encoded by an exogenous DNA. An example thereof is a gene associated with balance adjustment of protein production, such as the endoplasmic reticulum stress response gene; i.e., the activating transcription factor 4 (ATF4). The examples provided herein demonstrate that antibody production is enhanced when the Chinese hamster artificial chromosome of the present invention loaded with ATF4 in addition to the antibody gene. Other examples include genes known to be involved in material transportation between the endoplasmic reticulum (ER) and the Golgi body; i.e., the KDEL endoplasmic reticulum protein retention receptor 1 (KDELR1) gene, genes involved in expression control of the inflammatory secondary response gene; i.e., the nuclear factor kappa B inhibitor zeta (NFKBIZ) gene, transcription factors important for lipid biosynthesis; i.e., the sterol regulatory element binding protein 1 (SREBF1), and fatty acid desaturase; i.e., the stearoyl CoA desaturase 1 (SCD1) gene.

An example of another functional nucleic acid that can be used is a gene encoding a factor that is essential for host mammalian cell survival into which the Chinese hamster artificial chromosome vector of the present invention or a fragment thereof is transferred. In such a case, a factor that is essential for survival may be a gene encoding a protein or a gene in a noncoding region.

A mammalian cell used in such a case is preferably a cell that lacks endogenous expression of a factor that is essential for survival. A commercially available mammalian cell that lacks endogenous expression of a factor that is essential for survival may be purchased or such mammalian cell can be prepared by a technique known in the art, such as genome editing.

The mammalian cell that lacks endogenous expression of a factor that is essential for survival can survive only when the Chinese hamster artificial chromosome vector of the present invention or a fragment thereof comprising a gene encoding such factor is introduced thereinto. In contrast, a mammalian cell that does not have the Chinese hamster artificial chromosome vector of the present invention or a fragment thereof (e.g., a mammalian cell from which the artificial chromosome of the present invention or a fragment thereof has been deleted) cannot survive. This indicates that only the cell line that stably retains the Chinese hamster artificial chromosome or a fragment thereof is selected and such cell line, and it assures stable expression of a target protein that co-loaded on the Chinese hamster artificial chromosome or a fragment thereof. As described above, adoption of a system to select a cell comprising the Chinese hamster artificial chromosome vector of the present invention or a fragment thereof leads to improvement in productivity of a protein encoded by an exogenous DNA.

A specific example of a factor that is essential for mammalian cell survival is glutamine synthetase. Glutamine synthetase is essential for mammalian cell survival. Thus, a mammalian cell that lacks glutamine synthetase cannot survive, unless L-glutamine is supplied thereto from the outside with the aid of the Chinese hamster artificial chromosome vector of the present invention or a fragment thereof. Examples of other genes include the gene of dihydrofolate reductase (DHFR), the gene of pyrroline-5-carboxylase synthetase (P5CS), which is an enzyme catalyzing a reaction in the rate-determining step of proline biosynthesis, the gene of uridine monophosphate synthetase (UMPS), which is the gene of a multifunctional enzyme in nucleic acid biosynthesis, 5-aminoimidazole-4-carboxamide ribonucleotide formyltransferase/IMP cyclohydrolase (ATIC), which is the bifunctional purine biosynthesis protein gene, and the gene of arginase, which is an enzyme that constitutes the ornithine pathway involved in ammonia detoxication.

In addition, inhibition of expression of a particular gene may result in enhanced protein production, and a functional nucleic acid having such functions can be used in the present invention. Such functional nucleic acid inhibits particular biological functions, such as enzyme functions or catalyst functions, or perform biological inhibition (e.g., inhibition of transcription or translation) within an organism or cell, and preferably within a cell. Specifically, an RNA interference agent can be used, for example. Specific examples thereof include siRNA (small interfering RNA), shRNA (short hairpin RNA), and miRNA (micro RNA) (including pri-miRNA and pre-miRNA). Such functional nucleic acid can inhibit expression of, for example, a gene associated with homeostasis maintenance; i.e., BRCA1 (breast cancer 1).

### 3. A mammalian-derived cell comprising the Chinese hamster artificial chromosome vector of the present invention or a fragment thereof

The present invention provides a mammalian-derived cell comprising the Chinese hamster artificial chromosome vector or a fragment thereof described above.

The "mammalian-derived cells" include, but are not limited to, cells derived from primates such as human, monkey, and chimpanzee, rodents such as Chinese hamster, mouse, rat, hamster, and guinea pig, and ungulates such as cow, pig, sheep, and goat herein. In the present invention, mammalian-derived cells are preferably rodent-derived cells and primate-derived cells. In addition, it is particularly preferable that rodent-derived cells be Chinese hamster-derived cells and primate-derived cells be human-derived cells. As described above, cells derived from Chinese hamster ovary (CHO) are extensively used for production of useful proteins, such as antibody medicine.

When the Chinese hamster artificial chromosome vector of the present invention or a fragment thereof is introduced into a mammalian-derived cell (e.g., a cell derived from a rodent such as Chinese hamster), the Chinese hamster artificial chromosome vector or a fragment thereof will be stably retained in the mammalian-derived cell over a long period of time. When the Chinese hamster artificial chromosome vector of the present invention or a fragment thereof is "stably retained," the chromosome vector or a fragment thereof is less likely to be lost during cell division, i.e., the chromosome vector or a fragment thereof is stably retained in cells even after cell division, and, accordingly, the chromosome vector or a fragment thereof is efficiently transmitted to daughter cells or offspring mammals. The examples below demonstrate that CHO cells comprising the Chinese hamster artificial chromosome comprising a fluorescent gene as an exogenous gene introduced thereinto maintain 90% or more of the vector even after 25 times of passage culture (25 PDL).

A period defined by the term "long-term" or "a long period of time" herein is not particularly limited, and it indicates 10 times of passage culture, preferably 25 times of passage culture, more preferably 50 times of passage culture, and further preferably 70 times of passage culture. When the Chinese hamster artificial chromosome vector of the present invention or a fragment thereof is "stably retained," 80% or more, preferably 90% or more, more preferably 95% or more, and further preferably 99% or more thereof is maintained in the mammalian-derived cell after times of passage culture over a long period of time.

As described below, the Chinese hamster artificial chromosome vector of the present invention or a fragment thereof may be retained independently in a mammalian-derived cell, or it may be translocated to and retained in the chromosome of the mammalian-derived cell.

The term "translocate (or translocation)" used herein is as usually used in the art, and the term indicates that a given chromosome or a part thereof changes its position, for example, by adhering to another chromosome or other means. Specifically, the Chinese hamster artificial chromosome vector of the present invention or a fragment thereof may be translocated to and present in the chromosome of the host mammalian-derived cell. Through such translocation, stability of the artificial chromosome vector of the present invention or a fragment thereof can be improved with the aid of the original stability of the host chromosome in the cell. In the examples below, the Chinese hamster artificial chromosome vector translocated to the host chromosome is actually observed.

The Chinese hamster artificial chromosome vector of the present invention or a fragment thereof undergoes structural changes. For example, it may become a giant chromosome and may be maintained in the mammalian cell in that state. The Chinese hamster artificial chromosome of the present invention or a fragment thereof becomes to be giant when, for example, the number of gene expression units associated with high-level protein production or the number of target protein expression units is multiplied; i.e., multiple copies thereof are made, in any gene loading site of the Chinese hamster artificial chromosome of the present invention or a fragment thereof. When the artificial chromosome becomes giant, the number of gene copies is also increased, and it thus contributes to enhanced expression of the target protein. Gene amplification is verified based on the finding obtained by conventional gene amplification techniques, such as the MTX_DHFR system or the IR-MAR system. In the examples below, a giant Chinese hamster artificial chromosome vector is actually observed.

As a means for introducing the Chinese hamster artificial chromosome vector of the present invention or a fragment thereof into a mammalian-derived cell, various methods are known in the art. Examples of such methods include, but are not limited to, lipofection, a calcium phosphate method, microinjection, electroporation, and the microcell-mediated chromosome transfer (MMCT) method.

In addition, the Chinese hamster artificial chromosome vector of the present invention or a fragment thereof exhibits behavior independently of the mammalian-derived cell into which the vector or a fragment thereof has been introduced. Specifically, the Chinese hamster artificial chromosome vector or a fragment thereof introduced into the mammalian-derived cell can be transferred into another mammalian-derived cell using the means for introducing the artificial chromosome vector or a fragment thereof. The examples below demonstrate that a Chinese hamster artificial chromosome vector comprising a fluorescent protein gene can be transferred from a CHO cell to HT1080 derived from human fibrosarcoma. The MMCT method enables introduction of the Chinese hamster artificial chromosome vector of the present invention or a fragment thereof into any mammalian-derived cell.

The 3 types of the Chinese hamster cells comprising the he Chinese hamster artificial chromosome vector of the present invention were deposited by the applicant of the present invention internationally at the Patent Microorganisms Depositary, the National Institute of Technology and Evaluation (NITE) (122, 2-5-8 Kazusakamatari, Kisarazu-shi, Chiba, 292-0818, Japan) on January 6, 2022 under CHO(hprt-/-)CHAC C4 #2 (NITE BP-03585), CHO(hprt-/-)CHAC C4 #4 (NITE BP-03586), and CHO(hprt-/-)CHAC L14 #5 (NITE BP-03587). The method for preparing these deposited cells and functions thereof are described in detail in the examples below.

### 4. A method for producing a protein

The present invention provides a method for producing a protein. The method comprises: a step of production comprising culturing the cells described in 3. above to produce a protein encoded by an exogenous DNA; and a step of collection comprising collecting the protein.

Examples of the proteins that are produced herein include the industrially useful proteins and polypeptides in the fields of medicine, diagnosis, and agriculture. Specific examples are proteins or polypeptides encoded by the exogenous DNAs described in 2. above. The exogenous DNA is introduced into the Chinese hamster artificial chromosome vector or a fragment thereof to transfect the mammalian-derived cells. The thus-obtained cells are cultured, the exogenous DNAs are expressed to produce proteins or polypeptides, and the resulting proteins or polypeptides are collected from cells or a medium.

Culture conditions including a medium are selected depending on cell types, and known culture conditions can be employed. Examples of the media for animal cells include MEM medium, DMEM, Ham's F12 medium, Eagle's MEM medium, Iscove's EME medium, RPMI 1640 medium, and a mixture thereof.

Proteins or polypeptides may be collected (or isolated) by performing conventional techniques: for example, chromatography techniques, such as gel filtration chromatography, ion exchange chromatography, affinity chromatography, HPLC, and FPLC; salting-out; ammonium sulfate precipitation; organic solvent precipitation; ultrafiltration; crystallization; etc. Such techniques may be performed alone or in combination.

As described in 3. above, the Chinese hamster artificial chromosome vector of the present invention or a fragment thereof can be retained independently in a mammalian-derived cell or translocated to and retained in the chromosome of the mammalian-derived cell. Alternatively, the Chinese hamster artificial chromosome vector of the present invention or a fragment thereof can be maintained in the mammalian cell in the giant or other form.

Specifically, the Chinese hamster artificial chromosome vector of the present invention or a fragment thereof may be in any form. For example, it may be independent of a host mammalian-derived cell, it may be translocated to the chromosome of a mammalian-derived cell, or it may have become giant. In the process of genetic engineering for introducing an exogenous DNA into a mammalian cell, an artificial chromosome vector can undergo a variety of changes in its form, and, as a consequence, the artificial chromosome vector may be stabilized. Such changes in the form may increase exogenous DNA expression and enhance productivity of the target protein.

The Chinese hamster artificial chromosome vector of the present invention or a fragment thereof can further comprise a functional nucleic acid, in addition to an exogenous DNA. This can enhance productivity of a protein encoded by the exogenous DNA in the mammalian-derived cell comprising the Chinese hamster artificial chromosome vector of the present invention or a fragment thereof.

Such functional nucleic acid is as described in detail in 2. above. Specifically, a gene that accelerates exogenous DNA expression in a mammalian-derived cell can be used. Specific examples thereof include a gene associated with balance adjustment of protein production, such as the endoplasmic reticulum response gene; i.e., the activating transcription factor 4 (ATF4), the KDEL endoplasmic reticulum protein retention receptor 1 (KDELR1) gene, which is known to be involved in material transportation between the endoplasmic reticulum (ER) and the Golgi body, the nuclear factor kappa B inhibitor zeta (NFKBIZ) gene, which is involved in expression control of the inflammatory secondary response gene, the sterol regulatory element binding protein 1 (SREBF 1), which is a transcription factor important for lipid biosynthesis, and the stearoyl CoA desaturase 1 (SCD1) gene, which is fatty acid desaturase. It is also possible to accelerate exogenous DNA expression by suppressing expression of the endogenous gene in the host with the use of a functional nucleic acid. Examples of such functional nucleic acids include shRNA and miRNA targeting a gene associated with maintenance of homeostasis; i.e., BRCA1 (breast cancer 1).

Another example of a functional nucleic acid is a gene encoding a factor that is essential for survival of the host mammalian cell into which the Chinese hamster artificial chromosome vector of the present invention or a fragment thereof is transferred. In the mammalian cell that lacks endogenous expression of a factor that is essential for survival, a cell having the Chinese hamster artificial chromosome vector of the present invention or a fragment thereof comprising the gene encoding such factor can selectively survive. As described in 2. above, specific examples thereof include glutamine synthetase, dihydrofolate reductase (DHFR), pyrroline-5-carboxylase synthetase (P5CS), which is an enzyme catalyzing a reaction in the rate-determining step of proline biosynthesis, uridine monophosphate synthetase (UMPS), which is the gene of a multifunctional enzyme in nucleic acid biosynthesis, 5-aminoimidazole-4-carboxamide ribonucleotide formyltransferase/IMP cyclohydrolase (ATIC), which is the bifunctional purine biosynthesis protein gene, and the gene of arginase, which is an enzyme that constitutes the ornithine pathway involved in ammonia detoxication.

As described in 2. above, a vector for introducing an exogenous DNA comprising an exogenous DNA is transfected, so as to introduce the vector for introducing an exogenous DNA in a site-directed manner into the DNA insertion site of the mammalian artificial chromosome. When such transfection is performed in the present invention, the exogenous DNA loaded on the Chinese hamster artificial chromosome vector or a fragment thereof is subjected to gene amplification, and the protein productivity is thus enhanced. Such gene amplification can be achieved by gene introduction or with the use of an induction system.

When "the exogenous DNA is amplified by gene introduction," gene amplification is achieved without any artificial procedure when transfecting a vector for introducing an exogenous DNA comprising the Chinese hamster artificial chromosome. Such amplification can be achieved by, for example, the rearrangement described above or the giant CHAC described in 3. above that causes the formation of multiple copies whereby the number of gene expression units associated with high-level protein production or the number of target protein expression units is multiplied in any gene loading sites. While the protein productivity is enhanced as a consequence, a mode of gene amplification is not limited thereto.

When "the exogenous DNA is amplified with the use of an induction system," DNA constituting a known gene amplification system, such as the MTX_DHFR system or the IR-MAR system, is introduced together with the exogenous DNA into the Chinese hamster artificial chromosome of the present invention, so as to cause artificial gene amplification.

### 5. A kit for preparing a Chinese hamster artificial chromosome vector

The present invention provides a kit for preparing a Chinese hamster artificial chromosome vector comprising: the Chinese hamster artificial chromosome or a fragment thereof described in 1. above; and a vector comprising one or a plurality of DNA insertion sites included in the Chinese hamster artificial chromosome.

The Chinese hamster artificial chromosome or a fragment thereof used in the kit of the present invention is as described in 1. above. Also, the term "one or a plurality of DNA insertion sites" is as described in 1. above.

The vector included in the kit of the present invention comprises "one or a plurality of DNA insertion sites" included in the Chinese hamster artificial chromosome or a fragment thereof. Thus, the vector can recognize the insertion site of the Chinese hamster artificial chromosome or a fragment thereof and introduce DNA thereinto in a site-directed manner.

The vector included in the kit of the present invention may or may not comprise an "exogenous DNA." The term "exogenous DNA" used herein refers to a DNA encoding a useful protein that is intended to be expressed, as described in 2. above. When the kit is commercialized while the vector included therein does not comprise an "exogenous DNA," a user of the kit can introduce an "exogenous DNA" of interest into the vector of the kit of the present invention.

The kit of the present invention can be commercialized in the form that the vector comprises an exogenous DNA encoding a protein having a high demand for its production, in addition to "one or a plurality of DNA insertion sites." In such a case, it is not necessary for a user to introduce an exogenous DNA into the vector.

It is possible to prepare the mammalian-derived cell described in 3. above comprising the Chinese hamster artificial chromosome vector or a fragment thereof with the use of the kit of the present invention. This enables preparation of the useful protein described in 4. above.

### 6. A method for preparing a mammalian artificial chromosome or a fragment thereof

The present invention provides a method for preparing a mammalian artificial chromosome or a fragment thereof. The mammalian artificial chromosome or a fragment thereof described herein comprises a centromere and a telomere derived from a mammalian chromosome, a part of the chromosome, and one or a plurality of DNA insertion sites. The method of the present invention comprises: (1) a step of constructing a vector for inserting DNA; (2) a step of introducing a DNA insertion site; and (3) a step of fragmenting a chromosome.

The term "mammalian" used herein refers to a primate, such as a human, monkey, or chimpanzee, a rodent, such as a Chinese hamster, mouse, rat, hamster, or guinea pig, and an ungulate, such as a cow, pig, sheep, or goat, although the mammalian is not limited thereto. While the Chinese hamster artificial chromosome vector is prepared in the examples below, it is possible to prepare an artificial chromosome derived from any of the various types of mammalians exemplified above or a fragment thereof by the method of the present invention.

### (1) A step of constructing a vector for inserting DNA

This step comprises constructing a vector for inserting DNA comprising one or a plurality of DNA insertion sites. The "vector for inserting DNA" is used to introduce "one or a plurality of DNA insertion sites" into a mammalian artificial chromosome or a fragment thereof herein. The term "one or a plurality of DNA insertion sites" used herein is as defined in 1. above. With the use of the vector for inserting DNA constructed in this step, a DNA insertion site can be introduced into the mammalian artificial chromosome or a fragment thereof. In the examples below, a vector comprising φC31 and Bxb1 as the DNA insertion sites is constructed.

### (2) A step of introducing a DNA insertion site

The vector for inserting DNA is transfected into a mammalian cell, so as to introduce the DNA insertion site into the chromosome of the mammalian cell or a fragment thereof. Examples of transfection means include, but are not limited to, a method involving the use of Lipofectamine, the calcium phosphate method, and electroporation. In the examples below, the vector is introduced randomly into the chromosome of CHO cells using Lipofectamine to prepare CHO K1 cells (φC31/Bx).

The conventional artificial chromosome HAC vectors or MAC vectors disclosed in Patent Literature 1 to Patent Literature 5 and the like are constructed by identifying and deleting particular regions of the human or mouse chromosomes, so as to suppress the influence of the endogenous genes and maintain the functions as the chromosomes. According to the method of the present invention, in contrast, a vector for inserting DNA comprising a DNA insertion site or a fragment thereof is introduced randomly into a mammalian cell, unlike a conventional method comprising deleting a particular region in a particular chromosome of a mammal.

In the examples below, the CHO(hprt-/-)CHO-K1 cell line is established from the CHO K1 cell comprising the vector introduced thereinto (φC31/Bx) using the drug resistance as the indicator. CHO(hprt-/-)CHO-K1 is the CHO-K1 cell line that lacks the house keeping gene; i.e., hypoxanthine phosphoribosyltransferase (Hprt). The Hprt-deficient strain would not die because of incorporation of 6-thioguanine for the metabolic reasons and would show resistance to 6-thioguanine. Use of the cell with a drug resistance indicator enables selection of a cell comprising the target DNA introduced thereinto.

### (3) A step of fragmentation

After the step of introduction, a step of fragmentation comprising transferring a chromosome comprising a DNA insertion site from the donor mammalian cell comprising the DNA insertion site to a recipient mammalian cell by, for example, the microcell-mediated chromosome transfer (MMCT) method, electroporation, or lipofection and fragmenting the chromosome is performed.

According to the microcell fusion technique, a cell capable of micronucleus formation comprising a mammalian artificial chromosome vector (a donor cell) is subjected to microcell fusion with the other cell of interest (a recipient cell) to transfer the vector into the other cell.

The cell capable of micronucleus formation is treated with a polyploid inducer (e.g., colcemid or colchicine) to form a multinucleated cell having micronuclei. Then, the cell is subjected to cytochalasin treatment to form microcells and then to fusion to a target cell. By the microcell-mediated chromosome transfer method, the chromosome is transferred to the recipient mammalian cell. During such treatment, chromosome fragmentation and gene reconstitution (rearrangement) take place, and the mammalian artificial chromosome is thus prepared.

In the microcell-mediated chromosome transfer method, micronucleus formation of the donor cell can be performed by long-term culture of animal cells in a medium containing a polyploid inducer, such as colcemid. The polyploid inducer has the ability to induce decondensation of the chromosome and remodeling of the nuclear envelop.

As a result of micronucleus formation, a cell comprising a small amount of the cytoplasm and a micronucleus comprising one or a small number of chromosomes; i.e., a microcell, is formed from a donor cell. Culture is performed under conditions for donor cell culture. In general, a medium for animal cell culture is used.

With the use of cytochalasin B, the multinucleated cell is denucleated. A culture solution containing the multinucleated cell is introduced into a centrifuge tube, cytochalasin B is added thereto, and centrifugation is then performed. The precipitated microcells are suspended in a serum-free medium and collected. Microcells can be purified by ultrafiltration. For example, microcells are successively filtered through a membrane with a pore diameter of 8 µm, a membrane with a pore diameter of 5 µm, and a membrane with a pore diameter of 3 µm.

Fusion between microcells and recipient cells is performed by culture while overlaying purified microcells on the recipient cells that had terminated culture before full confluency. Microcell fusion can be performed by a technique, such as selection of a drug-resistant strain. Such fusion can be performed by a conventional technique, such as the polyethylene glycol (PEG) method.

The microcell-mediated chromosome transfer method is a method that has already been established as a method for transferring a vector into another cell. For example, such microcell-mediated chromosome transfer method is disclosed (Koi et al., Jpn. J. Cancer Res., 80: 413-418, 1973). In the microcell-mediated chromosome transfer method, a nuclear chromosome is always degraded in the step of micronucleating the cell nucleus. In the present invention, the chromosome is degraded with the aid of spontaneous degradation of the nuclear chromosome described above. Thus, a mammalian cell artificial chromosome with a small nucleotide size is generated.

### 7. A method for preparing a mammalian artificial chromosome vector or a fragment thereof

The present invention provides a method for preparing a mammalian artificial chromosome vector or a fragment thereof comprising introducing an exogenous DNA into a mammalian cell artificial chromosome or a fragment thereof comprising a centromere and a telomere derived from a mammalian chromosome, a part of the chromosome, and one or a plurality of DNA insertion sites. The method for preparing a mammalian artificial chromosome vector or a fragment thereof comprises: (1) a step of constructing a vector for introducing an exogenous DNA; and (2) a step of introducing a vector for introducing an exogenous DNA.

### (1) A step of constructing a vector for introducing an exogenous DNA

A step of constructing a vector for introducing an exogenous DNA comprises constructing a vector comprising one or a plurality of DNA insertion sites and an exogenous DNA encoding a protein that is intended to be expressed. The term "exogenous DNA" used herein is as defined in 2. above. The "vector for introducing an exogenous DNA" is used to introduce such exogenous DNA herein.

### (2) A step of introducing a vector for introducing an exogenous DNA

A step of introducing a vector for introducing an exogenous DNA comprises transfecting a vector for introducing an exogenous DNA into a cell comprising a mammalian artificial chromosome comprising a centromere and a telomere derived from a mammalian chromosome, a part of the chromosome, and one or a plurality of DNA insertion sites transferred thereto or a fragment thereof to introduce the vector for introducing the exogenous DNA in a site-directed manner into the DNA insertion site of the mammalian artificial chromosome.

With the use of the prepared mammalian artificial chromosome vector comprising the exogenous DNA or a fragment thereof, a mammalian-derived cell comprising the mammalian artificial chromosome vector or a fragment thereof can be prepared. With the use of the mammalian-derived cell, in addition, a useful protein can be prepared.

The vector for introducing an exogenous DNA can further comprise another DNA insertion site in addition to one or a plurality of the DNA insertion sites. In the embodiment which comprises another DNA insertion site, another exogenous DNA can be introduced into such site. This enables addition of an exogenous DNA encoding the protein that is intended to be expressed.

As described above, a plurality of exogenous DNAs can be introduced with the use of the mammalian artificial chromosome vector of the present invention.

### Examples

The present invention is described in more detail with reference to the examples below, although the technical scope of the present invention is not limited to the examples.

Figure 1 shows an overall view of preparation of the Chinese hamster artificial chromosome (CHAC) vector. As shown in left upper portion of Figure 1, a cyclic vector, which is an artificial gene comprising the DNA insertion sites (hereafter, may be referred to as the "gene loading sites"); i.e., bacteriophages phi(φ)C31 (white triangle) and Bxb1 (black triangle), tandemly arranged and the hygromycin-resistant gene (Hyg) ligated to a site downstream of the PGK promoter, was prepared (φC31/Bxb1 platform: φC31/Bx). The cyclic vector retaining the gene loading sites (the vector for inserting DNA) was inserted into the Chinese hamster ovary (CHO) cells; i.e., the CHO K1 cells (RCB line), by random integration to prepare the CHO K1 cells (φC31/Bx). The CHO K1 cells (φC31/Bx) comprising the cyclic vector inserted therein were screened based on hygromycin resistance, and the insertion of the vector was verified by PCR analysis and FISH analysis.

The CHO K1 cells (φC31/Bx) were screened using drug resistance to 6-thioguanine as the indicator to establish the CHO(hprt-/-)CHO-K1 cell line (not shown in Figure 1). CHO(hprt-/-)CHO-K1 is a CHO-K1 cell line that lacks a house keeping gene; i.e., hypoxanthine phosphoribosyltransferase (Hprt), and exhibits resistance to 6-thioguanine.

The CHO(hprt-/-)CHO-K1 cell line obtained above was used as a donor cell, the chromosome thereof was transferred into the recipient cell line by the microcell-mediated chromosome transfer (MMCT) method, and such transfer was verified by PCR and FISH. During the process, chromosome fragmentation took place, and cells retaining CHAC that retains the gene loading sites and has a reduced nucleotide size of the chromosome were obtained (CHO(Hprt(-/-))CHAC). CHAC obtained herein has φC31 and Bxb1 derived from φC31/Bx.

Separately, an antibody gene expression vector into which the gene loading sites; i.e., bacteriophage phi(φ)C31 (white triangle) and the antibody gene (shaded square), had been introduced was prepared, and the vector was introduced into the CHAC-retaining CHO(hprt-/-) cell line to establish a cell line that expresses the antibody gene. Introduction of the antibody gene expression vector into CHAC was verified by FISH analysis. Also, the cell was confirmed to actually produce the antibody by ELISA.

In addition, CHAC was transferred from the CHO(hprt-/-)CHO-K1 cell line retaining CHAC to the human fibrosarcoma cell; i.e., the HT1080 cell line, by the MMCT method (HT1080CHAC). CHAC transfer into the HT1080 cell line was verified by PCR analysis and FISH analysis.

The present invention is described in greater detail with reference to the following examples.

### [Example 1] Construction of a cyclic vector retaining an acceptor site (a gene loading site: a DNA insertion site)

[A] An acceptor site was introduced into the CHO chromosome by random integration to prepare an artificial chromosome from the Chinese hamster chromosome comprising the acceptor site inserted thereinto. At the outset, a vector loaded with a unit that retains an acceptor site capable of loading any gene by recombination and a drug resistant gene capable of drug selection when it is introduced into a cell and integrated into a chromosome was constructed (i.e., a vector for inserting DNA).

An artificial gene comprising bacteriophage phiC31 (φC31) and Bxb1 attP sites tandemly arranged downstream of the mouse PGK promoter was synthesized (Figure 2). attP is a site that is recognized by integrases (recombinases) of phiC31 and Bxb1, which are recombination sites on the phage genome side.

Also, a gene comprising the hygromycin-resistant (HygR) gene connected to a site downstream of the PGK promoter was artificially synthesized (Figure 3). The AscI-AvrII fragment shown in Figure 3 was inserted into the AscI/NheI site of the vector shown in Figure 2 (Figure 4). The chicken β globin insulator fragment (HS4) (1.2 kb) was inserted into the PspOMI/SnaBI site of the vector shown in Figure 3 (Figure 5).

### [Example 2] Random insertion of an acceptor site into the CHO-K1 (RCB strain) host chromosome and verification thereof

[A] A CHO-K1 cell comprising an acceptor site and a drug resistant gene randomly inserted into the chromosome is obtained.

### [A.1] Transfection and cell culture

The CHO-K1 (RIKEN BRC; RCB0285) cell line was cultured in a medium prepared by adding 10% (v/v) fetal bovine serum (NICHIREI; 175012, hereafter referred to as "FBS") and 100 units/ml penicillin/streptomycin (FUJIFILM Wako Pure Chemical Corporation; 168-23191) to the Ham's-F12 medium (Nacalai Tesque, Inc.;17458-65).

The CHO-K1 cell line was cultured on a 12-well plate to 70% confluency. A vector for loading an acceptor site was introduced using the Lipofectamine LTX Reagent with PLUS Reagent (Thermo Fisher Scientific; 15338100) in accordance with the manufacturer's instructions. A vector for loading an acceptor site was subjected to gene introduction in the form of a cyclic vector and in the form of a linear vector cleaved with AhdI (New England BioLabs; R0584). The cell line was subjected to passage culture at a plurality of cell densities to 10-cm dishes 24 hours after gene introduction and to drug selection with 200 µg/ml hygromycin B (FUJIFILM Wako Pure Chemical Corporation; 085-06153) 24 hours thereafter. From among drug-resistant clones obtained by introducing the cyclic vector and the linear vector, 18 clones and 29 clones were selected, respectively, and subjected to the subsequent analysis. Cell culture was performed at 37°C in the presence of 5% CO₂.

### [A.2] Selection of a cell comprising an acceptor site introduced thereinto by PCR analysis

The PGK promoter attP and the HygR gene regions (the vector shown in Figure 5) introduced into the cell were detected by PCR using the primers pEXA2J2_F2 and pEXA2J2_R2 shown below. Genomic DNAs were extracted from the clones using the Puregene kit (Qiagene), and PCR was performed using KOD One (TOYOBO) polymerase and the Eppendorf Mastercycler nexus gradient thermal cycler at 95°C for 30 seconds followed by 35 cycles at 98°C for 10 seconds, 62°C for 5 seconds, and 68°C for 20 seconds. pEXA2J2_F2: 5'-ATAAGCGGGTAAAATCCCGACTATG-3' (SEQ ID NO: 1) pEXA2J2_R2: 5'-GCATTTGGCGTATCCACTGACTATC-3' (SEQ ID NO: 2)

As a result of introduction of the cyclic vector and the linear vector, 9 PCR-positive clones and 19 PCR-positive clones were obtained, respectively (Figure 6).

### [A.3] Mono-color FISH analysis of cell clones

The PCR-positive clones obtained above were subjected to FISH analysis in accordance with the method of Matsubara et al. (FISH test protocol, Shujunsha Co., Ltd., 1994). In order to perform detection based on the FITC signal (green), FISH analysis was performed using a biotin-labeled vector for loading an acceptor site as a probe and counterstaining the chromosome with DAPI (blue).

Since the FITC signal (indicated by an arrow in Figure 7) was detected on the CHO host chromosome, it was visually verified that the vector for loading an acceptor site was introduced into the CHO host chromosome. The results demonstrate that the CHO chromosome comprising the acceptor site loaded thereon was obtained. On the basis of the results of FISH analysis, clones comprising the acceptor site loaded on a site close to the telomere or centromere region on the chromosome and retaining the same CHO chromosome loaded on the acceptor site among identical clones were selected.

In the subsequent step, 4 clones; i.e., CHO(A)-Hyg-C#2, CHO(A)-Hyg-C#4, CHO(A)-Hyg-L#14, and CHO(A)-Hyg-L#24, were used to construct artificial chromosomes.

### [Example 3] Establishment of HPRT(-)CHO-K1

The Hprt(-)CHO-K1 cells were obtained by subjecting CHO-K1 cells to passage culture from a 10-cm dish to ten 10-cm dishes, initiating drug selection in a culture medium supplemented with 6-thioguanine (MERCK; A4660) at the same time, and performing drug selection for 18 days to establish drug-resistant clones. The established drug-resistant clones were cultured in a medium supplemented with 1×HAT (MERCK; H0262) for 11 days and confirmed to be HAT-sensitive. The growth rate of the Hprt(-)CHO-K1 cells was measured using the IncuCyte S3 Kinetic Live Cell Imaging System (Essen BioScience). Clone cells were seeded at 1 × 10³ cells/well to 3 wells of a 96-well plate and cultured for 6 days. Cell culture was performed at 37°C in the presence of 5% CO₂.

The Hprt(-)CHO-K1 cells were selected using 6-thioguanine to establish drug-resistant clones. Whether or not the established drug-resistant clones would be Hprt-negative (Hprt(-)) and HAT-sensitive was examined. The results demonstrate that cells were killed in the medium supplemented with HAT. Thus, the clones obtained by 6-thioguanine selection were verified to be Hprt-negative (Hprt(-)) and HAT-sensitive.

The growth rate of the clones was examined. As a result, the growth rate of CHO RCB(hprt-/-) #2 was found to be the highest. Accordingly, CHO RCB(hprt-/-) #2 was used as the Hprt(-) CHO-K1 cell in the subsequent experiment (Figure 8).

### [Example 4] Chromosome transfer by MMCT

[A] Concerning the cell line comprising the acceptor site randomly introduced into the chromosome, the Chinese hamster chromosome comprising the acceptor inserted therein is transferred to the chromosome of the Hprt(-)CHO-K1 cell.

### [A.1] Establishment of the Hprt(-)CHO-K1 cell loaded with the CHO chromosome comprising the acceptor site

The Hprt(-)CHO-K1 cells as donor cells were cultured in a culture dish to 70% confluency. The medium was then exchanged with a Ham's-F12 medium supplemented with 20% FBS, 100 units/ml penicillin/streptomycin, 200 µg/ml hygromycin B, and 0.1 µg/ml colcemid (SIGMA; D7385), and after 48 hours of cultivation, the cells were further cultured in Ham's-F12 medium supplemented with 20% FBS, 100 units/ml penicillin/streptomycin, 200 µg/ml hygromycin B, and 0.1µg/ml colcemid. The culture product was incubated overnight to form microcells. The culture solution was removed, a centrifuge flask was filled with a 10 µg/ml cytochalasin B (Sigma; C6762) solution preheated to 37°C, and centrifugation was then performed at 34°C and 8000 rpm for 1 hour. The microcells were suspended in a serum-free DMEM medium and purified through 8-µm, 5-µm, and 3-µm filters. The purified product was centrifuged at 2000 rpm for 10 minutes, the resultant was suspended in 2 ml of a 0.05 mg/ml PHA-P solution, the culture solution was removed therefrom, and the remnant was added to the Hprt(-)CHO-K1 cells as recipient cells that had reached confluency in a 6-cm culture dish. The cells were incubated for 15 minutes to allow the micronucleated cells to adhere to the Hprt(-)CHO-K1 cells. Thereafter, 1 ml of a PEG1000 (Wako) solution (which is prepared by completely dissolving 5 g of PEG1000 in 6 ml of a serum-free DMEM medium, and adding 1 ml of dimethyl sulfoxide, followed by sterilization by filtration) was subjected to fusion for precisely a minute. The resultant was washed 4 times with 5 ml of serum-free DMEM to remove PEG and the Hprt(-)CHO-K1 culture solution was then added. The cells were seeded on ten 10-cm cell culture dishes 24 hours later, 200 µg/ml hygromycin B and 6-thioguanine were added thereto, and selection culture was then performed for 10 days. The drug-resistant strains obtained were subjected to the subsequent analysis.

### [A.2] PCR analysis

The chromosome-transferred cell clones were determined under the PCR conditions as employed for selection of the CHO chromosome loaded with the acceptor site. The PGK promoter attP and the HygR gene regions (the vector shown in Figure 5) were detected using the primers pEXA2J2_F2 and pEXA2J2_R2 in the same manner. Genomic DNAs were extracted from the clones and PCR was performed using KOD One (TOYOBO) polymerase and the Eppendorf Mastercycler nexus gradient thermal cycler at 95°C for 30 seconds followed by 35 cycles at 98°C for 10 seconds, 62°C for 5 seconds, and 68°C for 20 seconds. As a result, PCR-positive clones retaining the chromosomes were obtained (Figure 9).

### [A.3] FISH analysis

The PCR-positive clones obtained above were subjected to FISH analysis in accordance with the method of Matsubara et al. (FISH test protocol, Shujunsha Co., Ltd., 1994). In order to perform detection based on the FITC signal (green fluorescence), FISH analysis was performed using a biotin-labeled vector for loading an acceptor site as a probe and counterstaining the chromosome with DAPI.

Since the chromosome labeled with the FITC signal (indicated by an arrow in Figure 10) was maintained independently of the host chromosome, transfer of the CHO chromosome loaded with the acceptor site into the Hprt(-)CHO-K1 cell was visually verified. After chromosome transfer, a CHO chromosome loaded with the acceptor site was found to have the size remaining unchanged (Figure 10A). Other CHO chromosomes loaded with the acceptor site were found to have the size being reduced (Figures 10B, 10C, and 10D), and these chromosomes were designated as the Chinese hamster artificial chromosomes (hereafter referred to as "CHACs"). This indicates that deletion of a chromosome region during the process of chromosome transfer enables preparation of a minichromosome retaining the gene loading site.

The results demonstrate that Hprt(-)CHO-K1 cells retaining CHAC loaded with the acceptor site introduced thereinto with the size thereof being significantly reduced, compared with that before transfer, were obtained. In the subsequent experiment, clones selected therefrom were used.

### [Example 5] Verification of an acceptor site

[A] Whether or not a gene of interest can be loaded on the recombination site of CHAC is examined.

### [A.1] Construction of a vector for verifying gene introduction (a vector for loading an exogenous DNA)

In order to detect gene introduction, the green fluorescent protein gene EGFP and the red fluorescent protein gene mCherry were artificially synthesized (Figure 11A). These genes were inserted into the NheI/NotI site (Figure 11B) of pcDNA3.1 (Invitrogen) to prepare cDNA3.1-EGFP and pcDNA3.1-mCherry (Figure 11C).

The MluI/NotI fragment comprising a region from the CMV promoter to the poly A signal was cleaved from the completed vector (Figure 12A) and loaded on the MluI/NotI site (Figure 12B) of the insulator vector (Figure 12C).

An integrase-mediated gene recombination cassette comprising the blasticidin-resistant (bsd) gene and the G418-resistant (neo) gene positioned downstream of the phiC31 and Bxb1 attB (the recombination sites on the bacterial genome side) sites was artificially synthesized. Figure 13A shows the phiC31-bsd cassette and the Bxb1-neo cassette.

The phiC31-bsd and Bxb1-neo AscI/AvrII fragments were inserted into the AscI/NheI site (Figure 13B) of the vector shown in Figure 12 to prepare a fluorescent gene vector for integrase-mediated cell introduction (phiC31-bsd-EGF and Bxb1-neo-mCherry) (Figure 13C).

### [A.2] Transfection and cell culture

The Hprt(-)CHO-K1 cell line retaining CHAC was cultured in a medium prepared by adding 10% (v/v) FBS, 100 units/ml penicillin/streptomycin, and 200 µg/ml hygromycin B to the Ham's-F12 medium.

The Hprt(-)CHO-K1 cell line retaining CHAC was cultured on a 12-well plate to 70% confluency. A fluorescent gene vector was introduced using the Lipofectamine LTX Reagent with PLUS Reagent in accordance with the manufacturer's instructions. The cell line was subjected to passage culture at a plurality of cell densities to 10-cm dishes 24 hours after gene introduction. The cells comprising phiC31-bsd-EGFP and Bxb1-neo-mCherry introduced thereinto were subjected to drug selection with 8 µg/ml blasticidin S (KNF; KK-400) or 800 µg/ml G418 (Promega; V7983) 24 hours thereafter, so as to establish drug-resistant clones.

### [A.3] PCR analysis of cell clones

Genomes were extracted from blasticidin-resistant clones of phiC31 and G418-resistant clones of Bxb1 exhibiting fluorescent EGFP and mCherry, and whether or not specific recombination had occurred was examined by PCR (Figure 14).

phiC31-mediated gene introduction on the 5' side was examined using the primers PGK5 and bsd200AS and Bxb1-mediated gene introduction thereinto was examined using the primers PGK5 and neo310AS. PCR was performed using KOD One (TOYOBO) polymerase and the Eppendorf Mastercycler nexus gradient thermal cycler at 95°C for 30 seconds followed by 35 cycles at 98°C for 10 seconds, 65°C for 5 seconds, and 68°C for 5 seconds.

phiC31-mediated gene introduction on the 3' side was examined using the primers flori_seq and BxblattP_Rv and Bxb1-mediated gene introduction thereinto was examined using the primers flori_seq and HS4_R2. PCR was performed using KOD One (TOYOBO) polymerase and the Eppendorf Mastercycler nexus gradient thermal cycler at 95°C for 30 seconds followed by 35 cycles at 98°C for 10 seconds, 62°C for 5 seconds, and 68°C for 5 seconds.
PGK5: 5'-AATGGAAGTAGCACGTCTCACTAGTCTC-3' (SEQ ID NO: 3)
Bsd200AS: 5'-GCGACGATACAAGTCAGGTTGCCAGCTGCC-3' (SEQ ID NO: 4)
Neo310AS: 5'-GGTAGCCAACGCTATGTCCTGATAGCGGTC-3' (SEQ ID NO: 5)
Flori_seq: 5'-TAAGGGATTTTGCCGATTTC-3' (SEQ ID NO: 6)
Bxb1attP_Rv: 5'-AGACAAACCACGAAGACACAGGTC-3' (SEQ ID NO: 7)
HS4_R2: 5'-TCCTTTGCAACCCAGGCCGTTC-3' (SEQ ID NO: 8)

### [A.4] Two-color FISH analysis of cell clones

The PCR-positive clones obtained above were subjected to FISH analysis in accordance with the method of Matsubara et al. (FISH test protocol, Shujunsha Co., Ltd., 1994). FISH analysis was performed using a digoxigenin-labeled vector for loading an acceptor site for detection with rhodamine (red) and a biotin-labeled fluorescent gene vector for detection with FITC (green) as probes and counterstaining the chromosomes with DAPI.

As a result of the FISH analysis, no FITC signal or rhodamine signal was detected on the host chromosomes other than CHAC, and the FITC signal and the rhodamine signal were detected in a CHAC-directed manner (Figure 15). On the basis of the results of FISH analysis, it was visually verified that phiC31-bsd-EGFP and Bxb1-neo-mCherry were not randomly inserted into the host chromosome but were inserted in a site-directed manner into the acceptor site on CHAC. Also, cells maintaining CHAC independently of the CHO host chromosome (Figures 15A, 15B) and cells maintaining CHAC translocated to the host chromosome were observed (Figure 15C).

The results of the PCR analysis and the FISH analysis demonstrate that the phiC31-bsd-EGFP and Bxb1-neo-mCherry vectors were loaded on site-directed recombination site of CHAC.

### [Example 6] Loading two-site-directed recombination sites

### [A] A gene is loaded on the second site of the two-site-directed recombination sites of CHAC to prepare CHAC loaded with genes on all recombination sites.

### [A.1] Transfection and cell culture

The Hprt(-)CHO-K1 cell line retaining CHAC loaded with phiC31-bsd-EGFP was cultured in a medium prepared by adding 10% (v/v) FBS, 100 units/ml penicillin/streptomycin, and 8 µg/ml blasticidin S to the Ham's-F12 medium.

The Hprt(-)CHO-K1 cell line retaining CHAC loaded with phiC31-bsd-EGFP was cultured on a 12-well plate to 70% confluency. The Bxb1-neo-mCherry vector was introduced using the Lipofectamine LTX Reagent with PLUS Reagent in accordance with the manufacturer's instructions. The cells were subjected to passage culture at a plurality of cell densities to 10-cm dishes 24 hours thereafter, the cells were then subjected to drug selection using 800 µg/ml G418 (Promega; V7983) 24 hours thereafter, and drug selection was performed for 7 days, so as to establish 25 EGFP- and mCherry-positive drug-resistant clones.

### [A.2] PCR analysis of cell clones

Genomes were extracted from EGFP- and mCherry-positive clones, and whether or not specific recombination had occurred was examined by PCR (Figures 16 and 17: schematic diagram of recombination and PCR analysis).

Gene introduction on the 5' side was examined using the primers PGK5 and neo3 10AS. PCR was performed using KOD One (TOYOBO) polymerase and the Eppendorf Mastercycler nexus gradient thermal cycler at 95°C for 30 seconds followed by 35 cycles at 98°C for 10 seconds, 65°C for 5 seconds, and 68°C for 5 seconds (PCR region (1): Figure 17A).

For long-range PCR from EGFP to mCherry on CHAC loaded on the 5' side, the primers EGFP_F and mCherry_R were used. PCR was performed using KOD One (TOYOBO) polymerase and the Eppendorf Mastercycler nexus gradient thermal cycler at 95°C for 30 seconds followed by a cycle at 98°C for 10 seconds, 68°C for 5 seconds, and 68°C for 2 minutes, a cycle at 98°C for 10 seconds, 67°C for 5 seconds, and 68°C for 2 minutes, a cycle at 98°C for 10 seconds, 66°C for 5 seconds, and 68°C for 2 minutes, a cycle at 98°C for 10 seconds, 65°C for 5 seconds, and 68°C for 2 minutes, a cycle at 98°C for 10 seconds, 64°C for 5 seconds, and 68°C for 2 minutes, a cycle at 98°C for 10 seconds, 63°C for 5 seconds, and 68°C for 2 minute, and 27 cycles of 98°C for 10 seconds, 62°C for 5 seconds, and 68°C for 2 minutes (PCR region (2): Figure 17B, left).

For long-range PCR from mCherry to the hygromycin-resistant gene on CHAC loaded on the 3' side, the primers mCherry_F and hyg899AS were used. PCR was performed using KOD One (TOYOBO) polymerase and the Eppendorf Mastercycler nexus gradient thermal cycler at 95°C for 30 seconds followed by a cycle at 98°C for 10 seconds, 68°C for 5 seconds, and 68°C for 1 minute and 30 seconds, a cycle at 98°C for 10 seconds, 67°C for 5 seconds, and 68°C for 1 minute and 30 seconds, a cycle at 98°C for 10 seconds, 66°C for 5 seconds, and 68°C for 1 minute and 30 seconds, a cycle at 98°C for 10 seconds, 65°C for 5 seconds, and 68°C for 1 minute and 30 seconds, a cycle at 98°C for 10 seconds, 64°C for 5 seconds, and 68°C for 1 minute and 30 seconds, a cycle at 98°C for 10 seconds, 63°C for 5 seconds, and 68°C for 1 minute and 30 seconds, and 27 cycles at 98°C for 10 seconds, 62°C for 5 seconds, and 68°C for 1 minute and 30 seconds (PCR region (3): Figure 17B, right).
PGK5: 5'-AATGGAAGTAGCACGTCTCACTAGTCTC-3' (SEQ ID NO: 3)
Neo310AS: 5'-GGTAGCCAACGCTATGTCCTGATAGCGGTC-3' (SEQ ID NO: 5)
EGFP_F: 5'-AGCTGACCCTGAAGTTCATCTGC-3' (SEQ ID NO: 9)
mCherry_R: 5'-AACTTGATGTTGACGTTGTAGGCG-3' (SEQ ID NO: 10)
mCherry_F: 5'-CATGGCCATCATCAAGGAGTTCATG-3' (SEQ ID NO: 11)
hyg899AS: 5'-CCGGATCGGACGATTGCGTCGCATCG-3' (SEQ ID NO: 12)

As a result of PCR analysis, the Bxb1-neo-mCherry vector was found to have been loaded on the CHAC vector loading phiC31-bsd-EGFP. The results demonstrate that it is possible to load genes on both the φC31 and Bxb1 attP sites of the CHAC vector. On the basis of the results, 3 PCR-positive clones were subjected to FISH analysis.

### [A.3] Two-color FISH analysis of cell clones

The PCR-positive clones obtained above were subjected to FISH analysis in accordance with the method of Matsubara et al. (FISH test protocol, Shujunsha Co., Ltd., 1994). FISH analysis was performed using a digoxigenin-labeled vector for loading an acceptor site for detection with rhodamine (red) and a biotin-labeled Bxb1-neo-mCherry vector for detection with FITC (green) as probes and counterstaining the chromosomes with DAPI.

As a result of the FISH analysis, no FITC signal or rhodamine signal was detected on the host chromosomes other than CHAC, and the FITC signal and the rhodamine signal were detected in a CHAC-directed manner (Figure 18). On the basis of the results, it was visually verified that the Bxb1-neo-mCherry vector was not randomly inserted into the host chromosome but was inserted in a site-directed manner into the acceptor site on CHAC. Also, cells maintaining CHAC independently of the CHO host chromosome (Figures 18A, 18B) and cells maintaining CHAC translocated to the host chromosome (Figure 18C) were detected.

The results of the PCR analysis and the FISH analysis demonstrate that the Bxb1-neo-mCherry vector was loaded on the site-directed recombination site of CHAC.

### [Example 7] Loading an antibody gene on CHAC and establishment of an antibody-expressing strain

### [A] Loading of an antibody gene on CHAC and establishment of a cell expressing an antibody gene

### [A.1] Construction of an antibody expression vector (a vector for introducing an exogenous DNA)

The pcDNA3.1_Ab_HL vector having the full-length heavy chain (SEQ ID NO: 13) and the light chain (SEQ ID NO: 14) of the AMIGO2 antibody gene was digested with MluI-AvrII and inserted into the MluI-AvrII site of inspB4ins2 (the upper diagram in Figure 19A). This vector was designated as inspB4ins2_Ab_HL (the lower diagram in Figure 19A).

The phiC31-bsd cassette and the Bxb1-neo cassette (see Figure 13A) were each inserted into the AscI-NheI site of inspB4ins2_Ab_HL to construct vectors and the resulting vectors were designated as phiC31-bsd-Ab_HL and Bxb1-neo-Ab_HL, respectively (Figure 19B).

### [A.2] Transfection and cell culture

The Hprt(-)CHO-K1 cell line retaining CHAC was cultured in a medium prepared by adding 10% (v/v) FBS, 100 units/ml penicillin/streptomycin, and 200 µg/ml hygromycin B to the Ham's-F12 medium.

The Hprt(-)CHO-K1 cell line retaining CHAC was cultured on a 12-well plate to 70% confluency. The phiC31-bsd-Ab_HL vector and the Bxb1-neo-Ab_HL vector were introduced using the Lipofectamine LTX Reagent with PLUS Reagent in accordance with the manufacturer's instructions. The cells were subjected to passage culture at a plurality of cell densities to 10-cm dishes 24 hours thereafter, the cells comprising phiC31-bsd-EGFP and Bxb1-neo-mCherry introduced thereinto were then subjected to drug selection with 8 µg/ml blasticidin S (KNF; KK-400) or 800 µg/ml G418 (Promega; V7983) 24 hours thereafter, and drug selection was performed for 9 days, so as to establish drug-resistant clones.

### [A.3] PCR analysis of cell clones

PCR analysis and clone selection were performed in the same manner as in [Example 5] [A.3]. Genomes were extracted from various blasticidin-resistant clones of phiC31 and G418-resistant clones of Bxb1, and whether or not specific recombination had occurred was examined by PCR.

phiC31-mediated gene introduction on the 5' side was examined using the primers PGK5 and bsd200AS and Bxb1-mediated gene introduction thereinto was examined using the primers PGK5 and neo310AS. PCR was performed using KOD One (TOYOBO) polymerase and the Eppendorf Mastercycler nexus gradient thermal cycler at 95°C for 30 seconds followed by 35 cycles at 98°C for 10 seconds, 65°C for 5 seconds, and 68°C for 5 seconds.

phiC31-mediated gene loaded on the 3' side was examined using the primers flori_seq and Bxb1attP_Rv and Bxb1-mediated gene introduction thereinto was examined using the primers flori_seq and HS4_R2. PCR was performed using KOD One (TOYOBO) polymerase and the Eppendorf Mastercycler nexus gradient thermal cycler at 95°C for 30 seconds followed by 35 cycles at 98°C for 10 seconds, 62°C for 5 seconds, and 68°C for 5 seconds.
PGK5: 5'-AATGGAAGTAGCACGTCTCACTAGTCTC-3'(SEQ ID NO: 3)
Bsd200AS: 5'-GCGACGATACAAGTCAGGTTGCCAGCTGCC-3'(SEQ ID NO: 4)
Neo310AS: 5'-GGTAGCCAACGCTATGTCCTGATAGCGGTC-3'(SEQ ID NO: 5)
Flori_seq: 5'-TAAGGGATTTTGCCGATTTC-3'(SEQ ID NO: 6)
Bxb1attP_Rv: 5'-AGACAAACCACGAAGACACAGGTC-3'(SEQ ID NO: 7)
HS4_R2: 5'-TCCTTTGCAACCCAGGCCGTTC-3'(SEQ ID NO: 8)

### [A.4] ELISA analysis of cell clones

The drug-resistant clones were verified to express antibodies by ELISA. Antibodies obtained from the culture supernatant of the drug-resistant clones were used. For culture supernatant, the drug-resistant clones were seeded on a 24-well plate, the medium was exchanged with a fresh medium at 70% confluency, and static culture was then performed for 3 days. Thereafter, the culture supernatant was collected and stored at 4°C before ELISA analysis. The ELISA plate (442404, Nunc Maxisorp, Thermo Fisher Scientific) comprising the antigen protein immobilized thereon was blocked with TBS-T (50 mM Tris-HCl (pH 8.0) (T1503, Sigma-Aldrich), 150 mM NaCl (195-01663, FUJIFILM Wako Pure Chemical Corporation), and 0.05% (v/v) Tween 20 (160-21211, FUJIFILM Wako Pure Chemical Corporation)) supplemented with 5% skimmed milk (232100, Difco). The culture supernatant subjected to four-step serial dilution with a dilution factor of one-fourth and the positive control antibodies adjusted to 2, 0.5, 0.125, and 0.03125 µg/ml were added to the ELISA plate, a 1/50000 dilution of the Goat anti-Human IgG-Heavy and Light Chain Antibody HRP conjugated (A80-119P, Bethyl) was added thereto, and the reaction was then allowed to proceed. o-Phenylenediamine (FUJIFILM Wako Pure Chemical Corporation, 160-11022) was added to develop color, and 1M H₂SO₄ (Nacalai Tesque, Inc., 95626-06) was added to terminate the reaction. The absorbance at 492 nm was measured using a microplate reader Epoch2 (Bio Tek).

The results of ELISA analysis demonstrate that, in particular, clones derived from PCR-positive cells comprising the genes introduced into the φC31 site (Figure 20A) and the Bxb1 site (Figure 20B) more sufficiently express antibodies, compared with the controls (Figure 20C). This indicates that antibody-expressing cells were obtained. The results demonstrate that the selected drug-resistant clones express antibodies.

### [A.5] Two-color FISH analysis of cell clones

The PCR- and ELISA-positive clones obtained above were subjected to FISH analysis in accordance with the method of Matsubara et al. (FISH test protocol, Shujunsha Co., Ltd., 1994). FISH analysis was performed using a digoxigenin-labeled vector for loading an acceptor site for detection with rhodamine (red) and a biotin-labeled phiC31-bsd-Ab_HL for detection with FITC (green) as probes and counterstaining the chromosomes with DAPI.

As a result of the FISH analysis, no FITC signal or rhodamine signal was detected on the host chromosomes other than CHAC, and the FITC signal and the rhodamine signal were detected in a CHAC-directed manner (Figure 21). On the basis of the FISH analysis, it was visually verified that an expression vector phiC31-bsd-Ab_HL or Bxb1-neo-Ab_HL was not randomly inserted into the host chromosome but was inserted in a site-directed manner into the acceptor site on CHAC. Also, cells maintaining CHAC independently of the CHO host chromosome (Figures 21A, 21B), cells maintaining CHAC translocated to the CHO host chromosome (Figure 21C), and cells maintaining CHAC independently of the CHO host chromosome but having become giant (Figure 21D) were observed.

The results of the PCR analysis and the FISH analysis demonstrate that the antibody expression vector was loaded with the site-directed recombination site of CHAC. As a result of ELISA, in addition, the antibody gene loaded on CHAC was found to be expressed as a functional protein.

### [Example 8] Stability test

### [A] Expression stability of CHAC and a gene loaded on CHAC is verified by long-term culture in CHO cells.

### [A.1] Cell culture

Two clones and 3 clones selected from among the Hprt(-)CHO-K1 cells loaded with CHAC comprising φC31-BS-EGFP and Bxb1-neo-mCherry were subjected to the long-term stability test. The cells were subjected to long-term culture from 0 PDL to 25 PDL in a culture solution supplemented with 8 µg/ml blasticidin S or 800 µg/ml G418 or a culture solution free thereof in accordance with types of the drug-resistant genes loaded.

### [A.2] Analysis of fluorescent gene expressing cells by FCM

Concerning the Hprt(-)CHO-K1 cells retaining CHAC loaded with fluorescent genes, 5 clones in total; i.e., 2 clones loaded with φC31-BS-EGFP on the φC31 site; and 3 clones loaded with Bxb1-neo-mCherry on the Bxb1 site, were subjected to long-term culture. In FCM analysis, the fluorescence positivity was analyzed using a flow cytometer (CytoFLEX S; Beckman Coulter, Inc.) to 0 PDL, by culture to 25 PDL with the addition of a selection drug, and by culture to 25 PDL without the addition of a selection drug. The cells were suspended in FCM buffer comprising 1× PBS(-) (Nacalai Tesque, Inc., 14249-95) supplemented with 5% FBS. DAPI was added to the final suspension to remove dead cells.

As a result, expression of the fluorescent genes was observed in 98% or more cells of all the 5 clones when cultured to 0 PDL and cultured to 25 PDL with the addition of a selection drug (Figure 22). When cultured to 25 PDL without the addition of a selection drug, 82% to 83% of the cells loaded with φC31-BS-EGFP and 89% to 91% of the cells loaded with Bxb1-neo-mCherry introduced thereinto were found to be fluorescence-positive cells.

### [A.3] Analysis of CHAC-retaining rate by FISH analysis

FISH analysis was performed in accordance with the method of Matsubara et al. (FISH test protocol, Shujunsha Co., Ltd., 1994).

The Hprt(-) CHO-K1 cells retaining CHAC loaded with the fluorescent gene were subjected to FISH analysis with the use of a rhodamine-labeled vector for loading an acceptor site as a probe and counterstaining the chromosome with DAPI. The CHAC-retaining rate was calculated by analyzing the rhodamine signal positivity rate of the interphase image of 100 cells.

As a result of FISH analysis, retention of CHAC was observed in 95% or more cells of all the 5 clones when cultured to 0 PDL and cultured to 25 PDL with the addition of a selection drug (Figure 23). When cultured to 25 PDL without the addition of a selection drug, retention of CHAC was observed in 85% to 90% of the cells loaded with phiC31-bsd-EGFP and in 81% to 90% of the cells loaded with Bxb1-neo-mCherry.

### [Example 9] Establishment of HT1080 cells retaining CHAC

### [A] Whether CHAC transfer into human fibrosarcoma cells would enable CHAC transfer into cells of different species is examined.

### [A.1] MMCT

The HT1080 cell line derived from human fibrosarcoma was cultured in a medium prepared by adding 10% (v/v) FBS and 100 units/ml penicillin/streptomycin in the D-MEM medium (Nacalai Tesque, Inc.;08459-64).

The HPRT(-)CHO-K1 cells retaining CHAC as donor cells were cultured in a culture dish to 70% confluency, the medium was exchanged with the Ham's-F 12 medium supplemented with 20% FBS, 100 units/ml penicillin/streptomycin, 200 µg/ml hygromycin B, 400 nM Paclitaxel (Funakoshi; AG-CN2-0045-M100), and 200 nM Reversine (Funakoshi; 10004412), and the culture product was incubated to form microcells. The culture solution was removed, a centrifuge flask was filled with a 10 µg/ml cytochalasin B solution preheated to 37°C, and centrifugation was then performed at 34°C and 8000 rpm for 1 hour. The microcells were suspended in a serum-free DMEM medium and purified through 8-µm, 5-µm, and 3-µm filters. Thereafter, the suspension was centrifuged at 2000 rpm for 10 minutes, the resultant was suspended in 2 ml of a 0.05 mg/ml PHA-P solution, the culture solution was removed therefrom, and the remnant was added to the HT1080 cells as recipient cells that had reached confluency in a 6-cm culture dish. The cells were incubated for 15 minutes to allow the micronucleated cells to adhere to the HT1080 cells. Thereafter, 1 ml of a PEG1000 (Wako) solution (which is prepared by completely dissolving 5 g of PEG1000 in 6 ml of serum-free DMEM medium, and adding 1 ml of dimethyl sulfoxide, followed by sterilization by filtration) was subjected to cell fusion for precisely a minute. The resultant was washed 4 times with 5 ml of serum-free DMEM to remove PEG and the HT1080 culture solution was then added. The cells were seeded on ten 10-cm cell culture dishes 24 hours later, 200 µg/ml hygromycin B was added thereto, and selection culture was then performed for 8 days. The 21 clones selected from among the drug-resistant strains obtained were subjected to the subsequent analysis.

### [A.2] PCR analysis

The chromosome-transferred cell clones were determined under the PCR conditions as employed for selection of CHAC. The PGK promoter attP and the HygR gene regions (the vector shown in Figure 4) were detected using the primers pEXA2J2_F2 and pEXA2J2_R2 in the same manner. Genomic DNAs were extracted from the clones and PCR was performed using KOD One (TOYOBO) polymerase and the Eppendorf Mastercycler nexus gradient thermal cycler at 95°C for 30 seconds followed by 35 cycles at 98°C for 10 seconds, 62°C for 5 seconds, and 68°C for 20 seconds.

With the use of the Furin Fw and Furin Rv primers that specifically amplify the CHO chromosome region, contamination caused by donor CHO cells was detected. PCR was performed using KOD One (TOYOBO) polymerase and the Eppendorf Mastercycler nexus gradient thermal cycler at 98°C for 30 seconds followed by 35 cycles at 98°C for 10 seconds, 62°C for 5 seconds, and 68°C for 1 second.

The results of PCR demonstrate that the CHAC vector is capable of chromosome transfer into HT1080 cells and that the donor cells do not cause contamination (Figure 24). The results demonstrate that the CHAC vector is capable of chromosome transfer into other cells. On the basis of the results, 12 clones were selected and subjected to FISH analysis.

### [A.3] Two-color FISH analysis

The PCR-positive clones obtained above were subjected to FISH analysis in accordance with the method of Matsubara et al. (FISH test protocol, Shujunsha Co., Ltd., 1994). FISH analysis was performed using an FITC-labeled vector for loading an acceptor site and rhodamine-labeled human Cot-1 (Thermo Fisher Scientific; 15279-011) as probes and counterstaining the chromosomes with DAPI. As a result, it was visually verified that the CHAC vector is maintained independently of the HT1080 host chromosome (Figure 25).

### [Example 10] Improvement in protein productivity by loading a functional nucleic acid into CHAC

[A] A functional nucleic acid ATF4 is additionally loaded on CHAC comprising an antibody gene loaded thereinon and improvement in protein productivity by CHAC modification is verified.

### [A.1] Construction of a vector for introducing the ATF4 gene

The sequence information of the gene encoding ATF4 of a Chinese hamster (Cricetulus griseus) was searched for from the National Center for Biotechnology Information (NCBI) and obtained from the nucleotide sequence under Accession Number: XM_007654285 (Figure 26A). A sequence was artificially synthesized by substituting adenine 93 of the 1,047-bp sequence encoding the ATF4 gene with guanine (Figure 26B, Figure 27A). The nucleotide sequence shown in Figure 26A and Figure 26B are shown as SEQ ID NO: 15 and SEQ ID NO: 16 in the sequence listing. The synthesized s_CgATF4 gene was digested with EcoRI and HindIII and inserted into the enzyme site of the expression vector pCMV-EHpA (Figure 27A, Figure 27B, Figure 27C). The constructed expression vector pCMV-ATF4 was digested with MluI and AvrII, and the CMV promoter-ATF4-poly-A region was inserted into the enzyme site of the insulator vector (Figure 28A, Figure 28B, Figure 28C). To the inspB4ins2-CMV-ATF4 vector, the Bxb1neo cassette was inserted in the same manner as shown in Figure 13 of Example 5 to prepare Bxb1-neo-CMV-ATF4 (Figure 28D).

### [A.2] Transfection and cell culture

On the previous day of transfection, the cell line retaining CHAC expressing the AMIGO2 antibody (Example 7) was seeded at 1 × 10⁵ cells on a 6-well plate. On the day of transfection, the Bxb1-neo-CMV-ATF4 vector was introduced with the use of Lipofectamine LTX Reagent with PLUS Reagent in accordance with the manufacturer's instructions. The cells were subjected to passage culture at a plurality of cell densities to 10-cm dishes 24 hours thereafter, the cells were then subjected to drug selection with 800 µg/ml G418 (V7983; Promega) 24 hours thereafter, and drug selection was performed for 10 days, so as to establish drug-resistant clones.

### [A.3] PCR analysis of cell clones

After transfection, the genomes were extracted from G418-resistant clones, and whether or not specific recombination had occurred was examined (Figure 29).

Gene introduction on the 5' side was examined using the primers PGK5 and neo3 10AS. PCR was performed using KOD One (TOYOBO) polymerase and the Eppendorf Mastercycler nexus gradient thermal cycler at 95°C for 30 seconds followed by 35 cycles at 98°C for 10 seconds, 65°C for 5 seconds, and 68°C for 5 seconds.

Gene introduction on the 3' side was examined using the primers flori_seq and HS4_R2. PCR was performed using KOD One (TOYOBO) polymerase and the Eppendorf Mastercycler nexus gradient thermal cycler at 95°C for 30 seconds followed by 35 cycles at 98°C for 10 seconds, 62°C for 5 seconds, and 68°C for 5 seconds. The sequences are the same as those described in [Example 5] [A.3].

### [A.4] Two-color FISH analysis

The PCR-positive clones obtained above were subjected to FISH analysis in accordance with the method of Matsubara et al. (FISH test protocol, Shujunsha Co., Ltd., 1994). FISH analysis was performed using an FITC-labeled vector for loading an acceptor site and rhodamine-labeled ATF4 expression vector as probes and counterstaining the chromosomes with DAPI.

As a result of the FISH analysis, no FITC signal or rhodamine signal was detected on the host chromosomes other than CHAC, and the FITC signal and the rhodamine signal were detected in a CHAC-directed manner (Figure 30). On the basis of the FISH analysis, it was visually verified that the ATF4 expression vector was not randomly inserted into the host chromosome but was inserted in a site-directed manner into the acceptor site on CHAC. Also, cells maintaining CHAC independently of the CHO host chromosome (Figure 30A), cells maintaining CHAC translocated to the CHO host chromosome (Figure 30B), and cells maintaining CHAC independently of the CHO host chromosome but having become giant (Figure 30C) were observed.

The results of the PCR analysis and the FISH analysis demonstrate that the ATF4 expression vector was loaded on the site-directed recombination site of CHAC.

### [A.5] ELISA analysis of cell clones

An increase in the antibody titer was examined using ATF4-expressing cell clones by ELISA. The ATF4-expressing cells and the control cells (parent strains comprising no ATF4 introduced thereinto) were seeded at 1.0 × 10⁵ cells on a 6-well plate (Day 0). The culture supernatant (500 µl) was collected on Day 3 and 500 µl of a fresh medium was added. On Day 4 and Day 5, the culture supernatants (500 µl each) were collected as samples. Thereafter, the culture supernatants were stored at 4°C before ELISA analysis (the ELISA procedure is as described in Example 5).

As a result of ELISA analysis of the sample of Day 5, an increase in the antibody titer was observed in the ATF4-expressing cells, compared with the control cells (Figure 31A). An increase in the antibody titer was found to be 1.2 to 1.4 times (Figure 31B).

### Industrial Applicability

The Chinese hamster artificial chromosome of the present invention that can introduce a foreign DNA encoding a target protein or the like in a site-directed manner into one or a plurality of DNA insertion sites is useful for stable production of the target protein in mammalian cells such as CHO cells. Accordingly, the Chinese hamster artificial chromosome of the present invention is useful for stable production of biopharmaceuticals such as human antibodies.

### Sequence Listing Free Text

SEQ ID NOs: 1 to 12: primers
SEQ ID NO: 13: the nucleotide sequence of the anti-AMIGO2 antibody heavy chain gene (full-length)
SEQ ID NO: 14: the nucleotide sequence of the anti-AMIGO2 antibody light chain gene (full-length)
SEQ ID NO: 15: the nucleotide sequence of the ATF4 gene (Accession Number: XM_007654285)
SEQ ID NO: 16: the nucleotide sequence of the ATF4 gene with adenine 93 being substituted with guanine

All publications, patents, and patent applications cited herein are incorporated herein by reference in their entirety.

## Claims

1. A Chinese hamster artificial chromosome or a fragment thereof comprising a centromere and a telomere derived from a Chinese hamster chromosome, a part of the chromosome, and one or a plurality of DNA insertion sites.

2. The Chinese hamster artificial chromosome or a fragment thereof according to claim 1, wherein the nucleotide size is 10 Mbp or smaller.

3. The Chinese hamster artificial chromosome or a fragment thereof according to claim 1 or 2, wherein a sequence other than the DNA insertion site is derived from a Chinese hamster chromosome.

4. The Chinese hamster artificial chromosome or a fragment thereof according to any one of claims 1 to 3, wherein the DNA insertion site comprises at least one sequence selected from the group consisting of Bxb1 attP and Bxb1 attB sequences, φC31 attB and φC31 attP sequences, R4 attB and R4 attP sequences, TP901-1 attB and TP901-1 attP sequences, an FRT sequence, and a loxP sequence.

5. The Chinese hamster artificial chromosome or a fragment thereof according to any one of claims 1 to 4, which further comprises a reporter gene and/or a selection marker gene.

6. A Chinese hamster artificial chromosome vector or a fragment thereof, which comprises an exogenous DNA introduced in a site-directed manner into the DNA insertion site of the Chinese hamster artificial chromosome according to any one of claims 1 to 5.

7. The Chinese hamster artificial chromosome vector or a fragment thereof according to claim 6, which comprises a plurality of exogenous DNAs.

8. The Chinese hamster artificial chromosome vector or a fragment thereof according to claim 6 or 7, which further comprises another DNA insertion site in addition to one or a plurality of the DNA insertion sites.

9. The Chinese hamster artificial chromosome vector or a fragment thereof according to any one of claims 6 to 8, wherein the exogenous DNA is human DNA.

10. The Chinese hamster artificial chromosome vector or a fragment thereof according to claim 9, wherein the exogenous DNA is a DNA of a gene or a chromosome locus.

11. The Chinese hamster artificial chromosome vector or a fragment thereof according to claim 9 or 10, wherein the exogenous DNA is a gene encoding an immunoglobulin heavy chain and/or light chain or DNA of the gene.

12. The Chinese hamster artificial chromosome vector or a fragment thereof according to any one of claims 6 to 10, wherein the exogenous DNA is a gene selected from the group consisting of genes encoding polypeptides, such as cytokines, hormones, growth factors, nutritional factors, hematopoietic factors, coagulation or hemolysis factors, G protein-coupled receptors, and enzymes; a gene used for treatment of a disease, such as tumor, muscular dystrophy, hemophilia, neurodegenerative disease, autoimmune disease, allergic disease, and genetic disease; and an immune system gene or DNA sequences, such as the T cell receptor (TCR) or the human leukocyte antigen (HLA).

13. The Chinese hamster artificial chromosome vector or a fragment thereof according to any one of claims 6 to 12, which further comprises a functional nucleic acid.

14. The Chinese hamster artificial chromosome vector or a fragment thereof according to claim 13, wherein the functional nucleic acid is a gene that accelerates expression of an exogenous DNA.

15. The Chinese hamster artificial chromosome vector or a fragment thereof according to claim 13, wherein the functional nucleic acid is a gene encoding a factor that is essential for mammalian cell survival.

16. A mammalian-derived cell comprising the Chinese hamster artificial chromosome vector or a fragment thereof according to any one of claims 6 to 15.

17. The cell according to claim 16, which is transferred into a mammalian-derived cell and stably retained in the mammalian-derived cell over a long period of time.

18. The cell according to claim 16, which is retained independently in a mammalian-derived cell or retained in the chromosome translocated to the mammalian-derived cell.

19. The cell according to any one of claims 16 to 18, wherein the mammalian-derived cell is a rodent-derived cell or a primate-derived cell.

20. The cell according to claim 19, wherein the rodent-derived cell is a Chinese hamster-derived cell.

21. The cell according to claim 19, wherein the primate-derived cell is a human-derived cell.

22. The cell according to claim 16, which is deposited under CHO(hprt-/-)CHAC C4 #2 (NITE BP-03585).

23. The cell according to claim 16, which is deposited under CHO(hprt-/-)CHAC C4 #4 (NITE BP-03586).

24. The cell according to claim 16, which is deposited under CHO(hprt-/-)CHAC L14 #5 (NITE BP-03587).

25. A method for producing a protein comprising: a step of production comprising culturing the cell according to any one of claims 16 to 24 to produce a protein encoded by the exogenous DNA; and a step of collection comprising collecting the protein.

26. The method according to claim 25, which improves the protein productivity by further inclusion of a functional nucleic acid.

27. The method according to claim 26, wherein the functional nucleic acid is a gene that accelerates expression of an exogenous DNA in the cell.

28. The method according to claim 26, wherein the functional nucleic acid is a gene encoding the factor that is essential for cell survival and only the cell having the artificial chromosome vector or a fragment thereof is able to survive.

29. The method according to claim 25, which improves productivity of the protein by subjecting an exogenous DNA introduced into the Chinese hamster artificial chromosome to gene amplification.

30. A kit for preparing a Chinese hamster artificial chromosome vector comprising: the Chinese hamster artificial chromosome or a fragment thereof according to any one of claims 1 to 5; and a vector comprising one or a plurality of DNA insertion sites included in the Chinese hamster artificial chromosome.

31. The kit according to claim 30, wherein the vector further comprises an exogenous DNA.

32. A method for preparing a mammalian artificial chromosome or a fragment thereof comprising a centromere and a telomere derived from a mammalian chromosome, a part of the mammalian chromosome, and one or a plurality of DNA insertion sites, comprising:
a step of constructing a vector for inserting DNA comprising constructing a vector comprising one or a plurality of the DNA insertion sites;
a step of introducing a DNA insertion site comprising transfecting the vector for inserting DNA into a mammalian cell to introduce the DNA insertion site into the chromosome of the mammalian cell; and
a step of fragmentation comprising transferring a chromosome comprising a DNA insertion site from a donor mammalian cell comprising the DNA insertion site after the step of introduction to a recipient mammalian cell by the microcell-mediated chromosome transfer (MMCT) method and fragmenting the chromosome.

33. A method for preparing a mammalian artificial chromosome vector or a fragment thereof comprising:
a step of constructing a vector for introducing an exogenous DNA comprising constructing a vector for introducing an exogenous DNA comprising one or a plurality of DNA insertion sites and an exogenous DNA; and
a step of introducing a vector for introducing an exogenous DNA comprising transfecting the vector for introducing an exogenous DNA into a cell into which a mammalian artificial chromosome comprising a centromere and a telomere derived from a mammalian chromosome, a part of the chromosome, and one or a plurality of DNA insertion sites has been transferred, so as to introduce the vector for introducing an exogenous DNA in a site-directed manner into the DNA insertion site of the mammalian artificial chromosome.

34. The method for preparing a mammalian artificial chromosome vector or a fragment thereof according to claim 33, wherein the vector for introducing an exogenous DNA further comprises another DNA insertion site in addition to one or a plurality of the DNA insertion sites.
